# EUROPEAN PATENT APPLICATION

(11) **EP 4 397 750 A1**
(43) Date of publication of application: **10.07.2024**
(21) Application number: 22864731.9
(22) Date of filing: 02.09.2022
(51) Int. Cl.: C12N 5/071, C12N 5/074, C12Q 1/02

(54) **METHOD FOR PRODUCING INNER EAR STRIA VASCULARIS MARGINAL CELLS, CHEMICAL AGENT ASSESSMENT METHOD, AND CELL CULTURE FOR ASSESSING CHEMICAL AGENT**

(30) Priority: 03.09.2021 JP 2021144125
(71) Applicant: The Kitasato Institute, Tokyo 108-8641 (JP)
(72) Inventor: SAEGUSA, Chika, Sagamihara-shi, Kanagawa 252-0373 (JP); FUJIOKA, Masato, Sagamihara-shi, Kanagawa 252-0373 (JP); OKANO, Hideyuki, Tokyo 160-8582 (JP)
(74) Representative: Regimbeau
(86) International application number: PCT/JP2022/033140
(87) International publication number: WO 2023/033149

(57) **Abstract**

The present invention provides a technology for creating functional inner ear stria vascularis marginal cells that are potentially applicable for screening of agents and pathological analysis of hearing impairment. A method for producing inner ear stria vascularis marginal cells includes a step of culturing a cell population including inner ear progenitor cells in an insulin-free culture medium that does not contain insulin or contains insulin only in a trace amount.

## Description

### [Technical Field]

The present invention relates to a method for producing inner ear stria vascularis marginal cells, an agent assessment method, and a cell culture for assessing an agent.

### [Background Art]

Hearing impairment is a disease that causes a significant decrease in QOL, but there is still no fundamental treatment method. In addition, hearing impairment has recently attracted attention as a risk factor for dementia and the like, and understanding the mechanism of the onset of hearing impairment and establishing a treatment method are important and urgent issues.

Conventionally, rodents such as genetically modified mice, and mice and rats administered with drugs have been mainly used as model animals for analyzing the mechanism of the onset of hearing impairment. However, many examples are reported in which the phenotype of the rodent model animal does not necessarily match the human pathophysiology, and the pathological analysis using human cells and tissues has been particularly important in recent years. However, it is difficult to anatomically and non-invasively collect the stria vascularis from a human as well as other inner ear tissues, and it is difficult to obtain human pathological tissues because hearing impairment itself is not a lethal disease. Therefore, it is not easy to analyze the mechanism of the onset of hearing impairment using human cells and tissues.

As a research tool for solving such problems, in recent years, pluripotent stem cell-derived differentiated cells such as embryonic stem cells (ES cells) and induced pluripotent stem cells (iPS cells) have been attracting attention. Also in the laboratory to which the present inventors belong, a method for inducing differentiation into inner ear cell-like cells from human iPS cells has been developed (Patent Document 1), a pathological analysis system has been constructed, and a search for a therapeutic drug for hearing impairment called Pendred syndrome (PDS) has been conducted using this method (Non-Patent Document 1). In addition, the effectiveness of some of the candidate drugs obtained as a result of the search has been confirmed in a physician-led clinical trial (clinical trial registration ID: UMIN000033083).

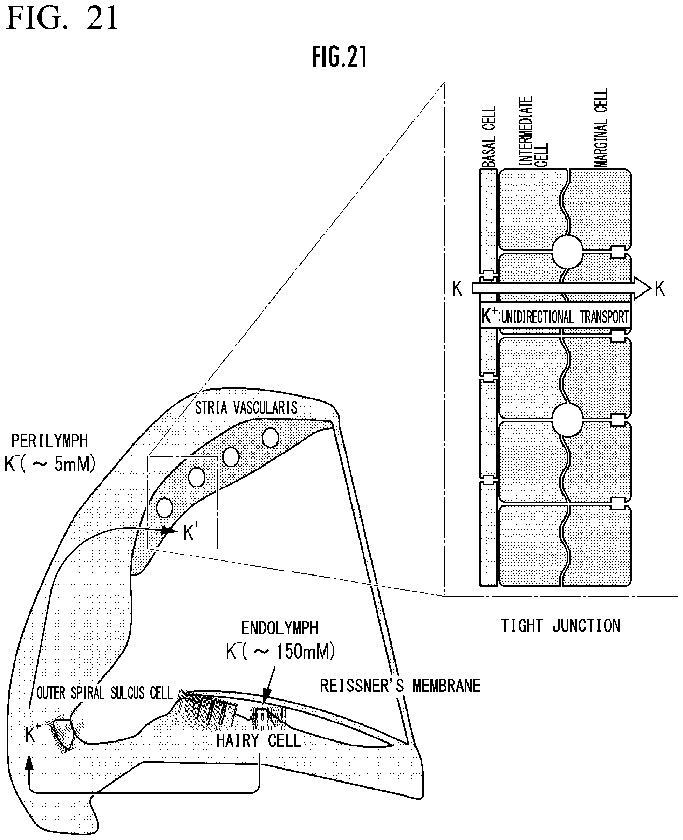

On the other hand, the inner ear cochlea stria vascularis is an essential tissue for maintaining the homeostasis of the ion environment in the inner ear cochlea (see left column of FIG. 21), and it is considered that one of the causes of many types of hearing impairment such as genetic, drug-induced, age-related, noise-induced, and viral hearing impairment is the dysfunction of the stria vascularis. Among the three types of cells constituting the stria vascularis, the marginal cells are cells having an essential function in unidirectional ion transport and barrier formation between the tissue and the endolymph (see right column of FIG. 21).

### [Citation List]

### [Non-Patent Document]

[Non-Patent Document 1]
Makoto Hosoya, Masato Fujioka, Takefumi Sone, Satoshi Okamoto, Wado Akamatsu, Hideki Ukai, Hiroki R. Ueda, Kaoru Ogawa, Tatsuo Matsunaga, and Hideyuki Okano, "Cochlear Cell Modeling Using Disease-Specific iPSCs Unveils a Degenerative Phenotype and Suggests Treatments for Congenital Progressive Hearing Loss", Cell Reports 18, 68-81, January 3, 2017.

### [Patent Document]

[Patent Document 1]
Japanese Patent (Granted) Publication No. 6218152

### [Summary of Invention]

### [Technical Problem]

In the marginal cell-like cells differentiation-induced in the related art, although the expression of some markers is recognized, the cobblestone-like planar structure by intercellular tight junction proteins required to maintain the homeostasis of the potassium ion concentration is not formed. Therefore, it is difficult to say that the cells exhibit the same physiological functionality as the stria vascularis marginal cells endogenous to the inner ear organ, and thus the cells have not been applied to the screening of agents and pathological analysis of hearing impairment in which the inner ear stria vascularis marginal cells are involved.

Therefore, an object of the present invention is to provide a technology for creating functional inner ear stria vascularis marginal cells that are potentially applicable for screening of agents and pathological analysis of hearing impairment.

### [Solution to Problem]

In order to achieve the aforementioned objects, the inventors of the present invention have repeated intensive studies and have completed the present invention.

That is, the present invention provides, in a first aspect, a method for producing inner ear stria vascularis marginal cells, the method including a step of culturing a cell population including inner ear progenitor cells differentiation-induced from pluripotent stem cells in an insulin-free culture medium that does not contain insulin or contains insulin only in a trace amount.

According to the method for producing inner ear stria vascularis marginal cells of the present invention, since the cell population including the inner ear progenitor cells differentiation-induced from pluripotent stem cells is cultured in an insulin-free culture medium that does not contain insulin or contains insulin only in a trace amount, functional inner ear stria vascularis marginal cells that are potentially applicable for screening of agents and pathological analysis of hearing impairment can be obtained.

In the method for producing inner ear stria vascularis marginal cells according to the present invention, the insulin-free culture medium preferably has an insulin concentration of 0 nM or more and 100 nM or less. According to this, it is possible to more reliably induce differentiation into inner ear stria vascularis marginal cells having good quality.

In the method for producing inner ear stria vascularis marginal cells according to the present invention, the insulin-free culture medium preferably contains EGF and further contains at least one or more selected from the group consisting of bFGF, FGF3, and BMP4. In addition, particularly in the suspension culture, the first insulin-free culture medium in the suspension culture period preferably contains EGF, FGF3, and BMP4. According to this, it is possible to more reliably induce differentiation into inner ear stria vascularis marginal cells having good quality.

In the method for producing inner ear stria vascularis marginal cells according to the present invention, the following steps (1) and (2) are preferably included.
(1) A step of subjecting the cell population including the inner ear progenitor cells differentiation-induced from the pluripotent stem cells to cell detaching and dispersing treatment
(2) A step of subjecting the cells or the cell population obtained in step (1) to suspension culture in the insulin-free culture medium in the presence of an extracellular matrix material

The extracellular matrix material is preferably at least one or more selected from the group consisting of Matrigel, Pronectin, collagen, laminin, and fibronectin.

According to the aforementioned production method, since the cell population including inner ear progenitor cells differentiation-induced from pluripotent stem cells is subjected to cell detaching and dispersing treatment, cells that are not suitable for culture are eliminated, and it is possible to more reliably obtain the inner ear progenitor cells having good quality. In addition, the suspension culture in the presence of the extracellular matrix material has an effect of serving as a scaffold for differentiation into stria vascularis marginal cells. Consequently, it is possible to more reproducibly obtain inner ear stria vascularis marginal cells having good quality.

In the method for producing inner ear stria vascularis marginal cells according to the present invention, the following steps (1) to (3) are preferably included.
(1) A step of subjecting the cell population including the inner ear progenitor cells differentiation-induced from the pluripotent stem cells to cell detaching and dispersing treatment
(2) A step of subjecting the cells or the cell population obtained in step (1) to suspension culture in the insulin-free culture medium in the presence of an extracellular matrix material
(3) A step of seeding the cells or the cell population after the suspension culture in step (2) on feeder cells that have been separately subjected to adhesion culture in advance, and culturing the cells or the cell population in the insulin-free culture medium

The feeder cells are preferably melanocytes or melanocyte-like cells.

According to the aforementioned production method, since the cell population including inner ear progenitor cells differentiation-induced from pluripotent stem cells is subjected to cell detaching and dispersing treatment, cells that are not suitable for culture are eliminated, and it is possible to more reliably obtain the inner ear progenitor cells having good quality. In addition, since the cells are subjected to suspension culture in the insulin-free culture medium in the presence of the extracellular matrix material, the presence of the extracellular matrix material has an effect of serving as a scaffold for differentiation into stria vascularis marginal cells. Furthermore, since the cells or the cell population after suspension culture are seeded on feeder cells that have been separately subjected to adhesion culture in advance, and cultured in an insulin-free culture medium, the formation of a structure (layered) in which the stria vascularis marginal cells are two-dimensionally cultured on the surface of the feeder cells is promoted also in the inner ear stria vascularis marginal cells co-cultured with the feeder cells subjected to adhesion culture. Consequently, it is possible to more reproducibly perform two-dimensional culture of inner ear stria vascularis marginal cells having good quality.

In the method for producing inner ear stria vascularis marginal cells according to the present invention, the cell population including the inner ear progenitor cells obtained by inducing differentiation from the pluripotent stem cells is preferably obtained by a method including the following steps (1) to (4).

(1) A step of culturing the pluripotent stem cells in the absence of a growth factor and in the presence of a ROCK inhibitor.
(2) A step of culturing the cell population obtained in step (1) in the absence of a growth factor and a ROCK inhibitor.
(3) A step of culturing the cell population obtained in step (2) in the presence of at least one growth factor selected from the group consisting of bFGF, FGF3, FGF10, and FGF19, and in the presence of BMP4.
(4) A step of culturing the cell population obtained in step (3) in the presence of at least one growth factor selected from the group consisting of bFGF, FGF3, FGF10, and FGF19, and in the absence of BMP4.

According to the aforementioned production method, it is possible to more reliably induce differentiation into inner ear progenitor cells from pluripotent stem cells, and consequently, it is possible to more reproducibly obtain inner ear stria vascularis marginal cells having good quality.

In the method for producing inner ear stria vascularis marginal cells according to the present invention, the inner ear stria vascularis marginal cells are preferably obtained under a serum-free condition. According to this, the risk of differentiation into unintended cells caused by serum factor can be suppressed.

In the method for producing inner ear stria vascularis marginal cells according to the present invention, the inner ear stria vascularis marginal cells preferably express a potassium channel protein and a tight junction protein. According to this, it is possible to more reliably ensure the quality of the obtained cells as functional inner ear stria vascularis marginal cells, and consequently, it is possible to more reproducibly obtain inner ear stria vascularis marginal cells having good quality.

The present invention provides, in a second aspect, an agent assessment method including a step of treating inner ear stria vascularis marginal cells that are obtained by inducing differentiation from inner ear progenitor cells with a test agent, and a step of assessing a state of the inner ear stria vascularis marginal cells treated with the test agent.

According to the agent assessment method of the present invention, it is possible to effectively and efficiently assess an agent that affects the inner ear stria vascularis marginal cells.

The present invention provides, in a third aspect, a cell culture for assessing an agent, including inner ear stria vascularis marginal cells that are obtained by inducing differentiation from inner ear progenitor cells.

According to the cell culture for assessing an agent of the present invention, since the cell culture includes the inner ear stria vascularis marginal cells that are obtained by inducing differentiation from inner ear progenitor cells, it is possible to effectively and efficiently assess an agent that affects the inner ear stria vascularis marginal cells by using the cell culture.

The present invention provides, in a fourth aspect, a method for producing inner ear stria vascularis marginal cells, the method including a step of culturing a cell population including inner ear progenitor cells in an insulin-free culture medium that does not contain insulin or contains insulin only in a trace amount.

According to the method for producing inner ear stria vascularis marginal cells of the present invention, since the cell population including the inner ear progenitor cells is cultured in an insulin-free culture medium that does not contain insulin or contains insulin only in a trace amount, functional inner ear stria vascularis marginal cells that are potentially applicable for screening of agents and pathological analysis of hearing impairment can be obtained.

### [Brief Description of Drawings]

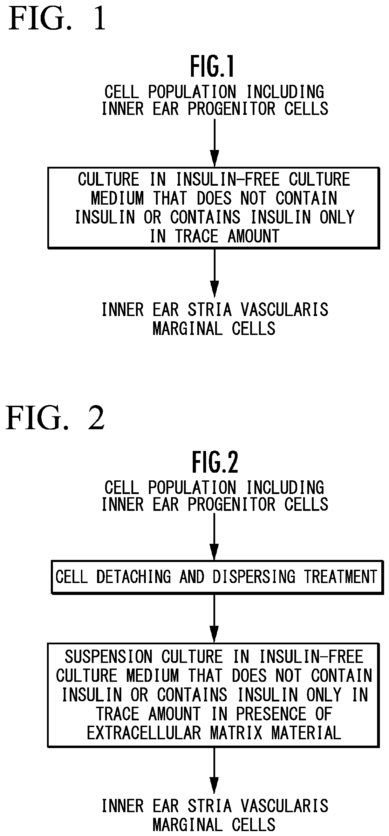

FIG. 1 is a flowchart representing one embodiment of a method for producing inner ear stria vascularis marginal cells according to the present invention.
FIG. 2 is a flowchart representing another embodiment of a method for producing inner ear stria vascularis marginal cells according to the present invention.

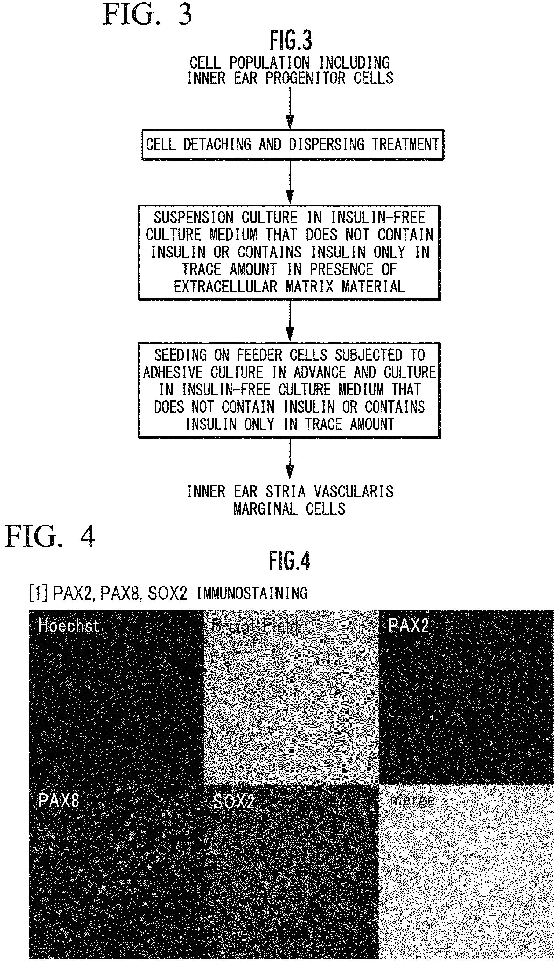

FIG. 3 is a flowchart representing still another embodiment of a method for producing inner ear stria vascularis marginal cells according to the present invention.
FIG. 4 is a diagram representing the results of, in Test Example 1, inducing differentiation from pluripotent stem cells into inner ear progenitor cells and detecting the expression of PAX2, PAX8, and SOX2, which are known marker molecules of inner ear progenitor cells, by immunostaining with each of the specific antibodies.

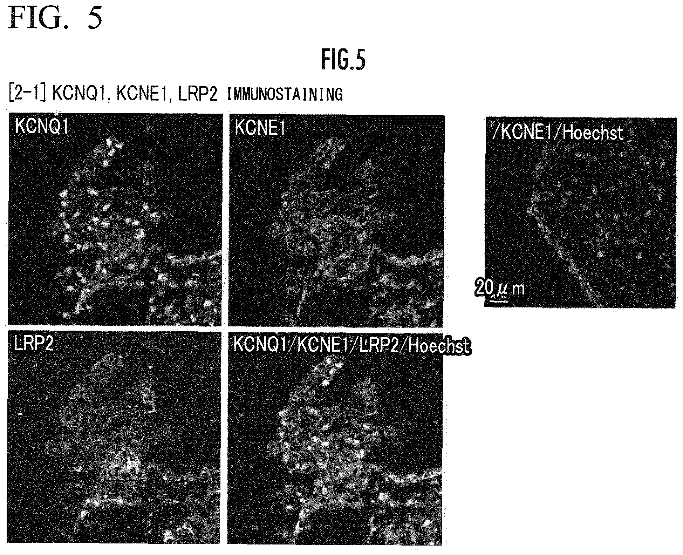

FIG. 5 is a diagram representing the results of, in Test Example 2, inducing differentiation from inner ear progenitor cells into inner ear stria vascularis marginal cells and detecting the expression of KCNQ1, KCNE1, and LRP2, which are known functional proteins in the inner ear stria vascularis marginal cells, by immunostaining with each of the specific antibodies.

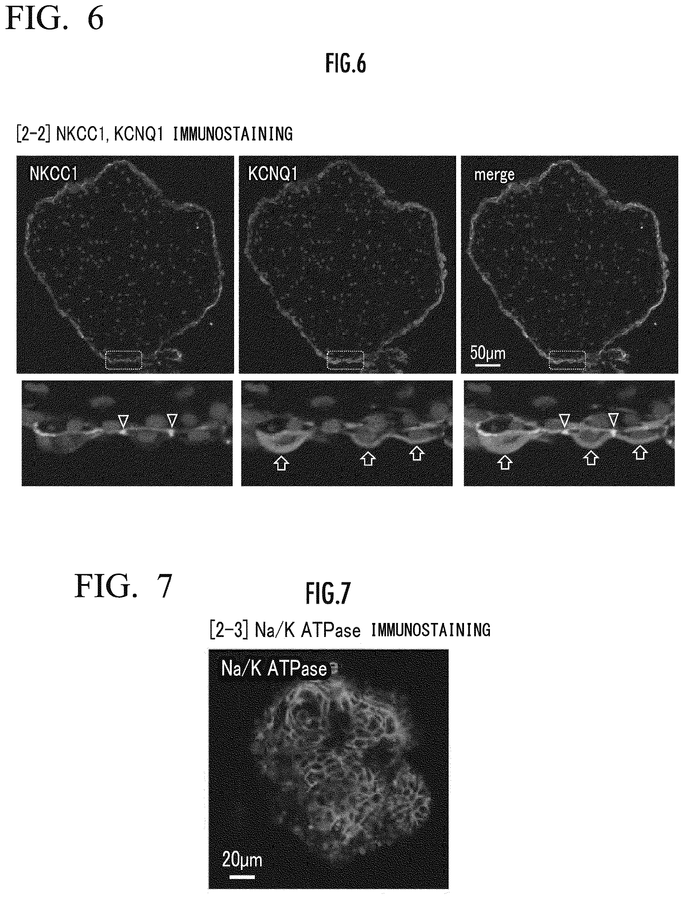

FIG. 6 is a diagram representing the results of, in Test Example 2, inducing differentiation from inner ear progenitor cells into inner ear stria vascularis marginal cells and detecting the expression of NKCC1 and KCNQ1, which are known functional proteins in the inner ear stria vascularis marginal cells, by immunostaining with each of the specific antibodies.
FIG. 7 is a diagram representing the results of, in Test Example 2, inducing differentiation from inner ear progenitor cells into inner ear stria vascularis marginal cells and detecting the expression of Na/K ATPase, which is a known functional protein in the inner ear stria vascularis marginal cells, by immunostaining with the specific antibody.

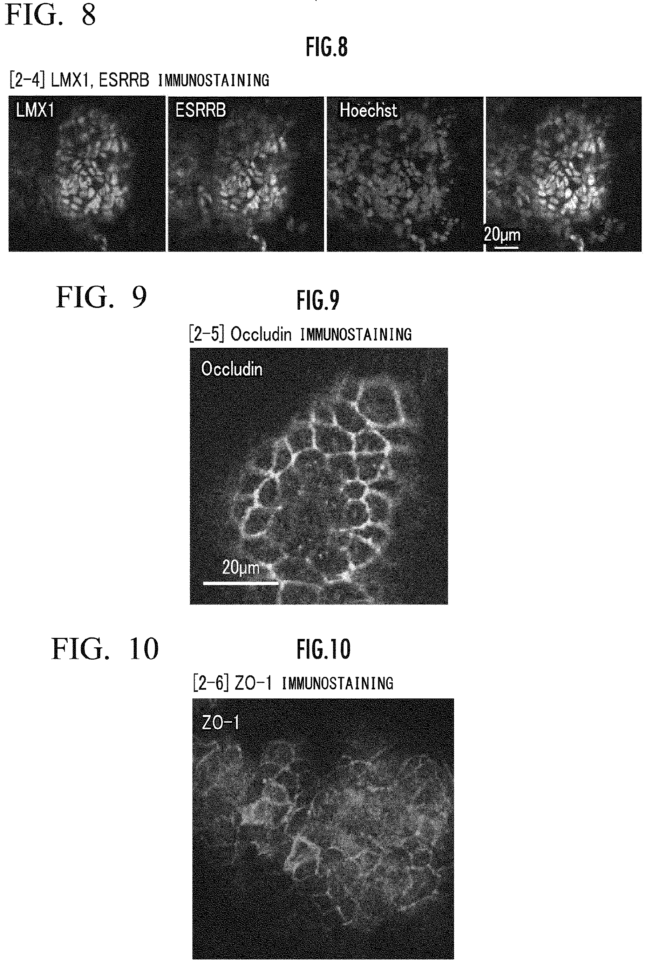

FIG. 8 is a diagram representing the results of, in Test Example 2, inducing differentiation from inner ear progenitor cells into inner ear stria vascularis marginal cells and detecting the expression of LMX1 and ESRRB, which are known functional proteins in the inner ear stria vascularis marginal cells, by immunostaining with each of the specific antibodies.
FIG. 9 is a diagram representing the results of, in Test Example 2, inducing differentiation from inner ear progenitor cells into inner ear stria vascularis marginal cells and detecting the expression of Occludin, which is a known intercellular tight junction protein in the inner ear stria vascularis marginal cells, by immunostaining with the specific antibody.
FIG. 10 is a diagram representing the results of, in Test Example 2, inducing differentiation from inner ear progenitor cells into inner ear stria vascularis marginal cells and detecting the expression of ZO-1, which is a known intercellular tight junction protein in the inner ear stria vascularis marginal cells, by immunostaining with the specific antibody.

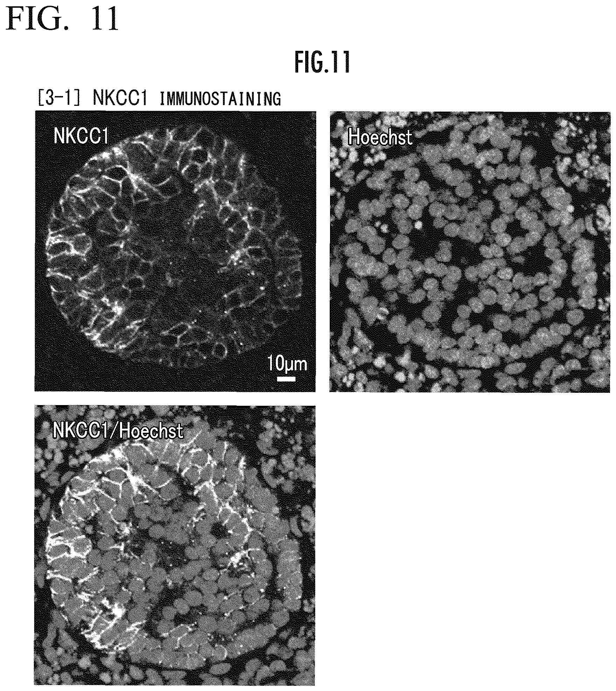

FIG. 11 is a diagram representing the results of, in Test Example 3, inducing differentiation into inner ear progenitor cells from pluripotent stem cells, inducing further differentiation into inner ear stria vascularis marginal cells, and detecting the expression of NKCC1, a known functional protein in the inner ear stria vascularis marginal cells, by immunostaining with the specific antibody.

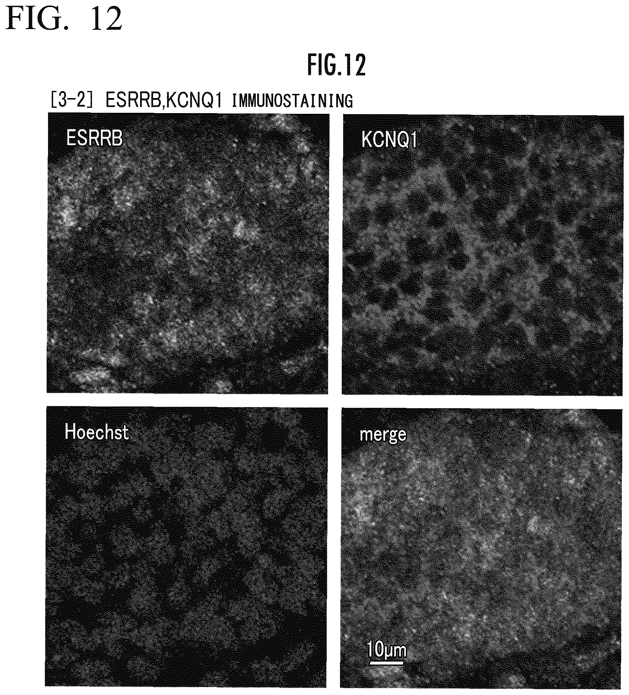

FIG. 12 is a diagram representing the results of, in Test Example 3, inducing differentiation into inner ear progenitor cells from pluripotent stem cells, inducing further differentiation into inner ear stria vascularis marginal cells, and detecting the expression of ESRRB and KCNQ1, known functional proteins in the inner ear stria vascularis marginal cells, by immunostaining with the specific antibody.

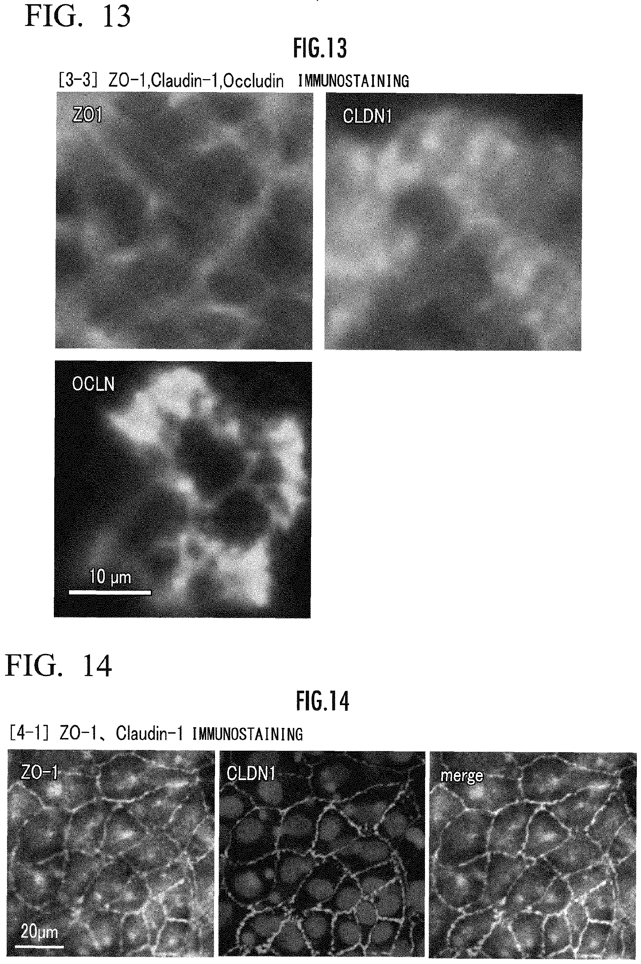

FIG. 13 is a diagram representing the results of, in Test Example 3, inducing differentiation into inner ear progenitor cells from pluripotent stem cells, inducing further differentiation into inner ear stria vascularis marginal cells, and detecting the expression of Occludin, Claudin-1, and ZO-1, which are known intercellular tight junction proteins in the inner ear stria vascularis marginal cells, by immunostaining with the specific antibody.
FIG. 14 is a diagram representing the results of, in Test Example 4, inducing differentiation from inner ear progenitor cells into inner ear stria vascularis marginal cells by co-culture with the feeder cells (melanocytes) that have been separately subjected to adhesion culture in advance, and detecting the expression of ZO-1 and Claudin-1, which are known intercellular tight junction proteins in the inner ear stria vascularis marginal cells, by immunostaining with each of the specific antibodies.

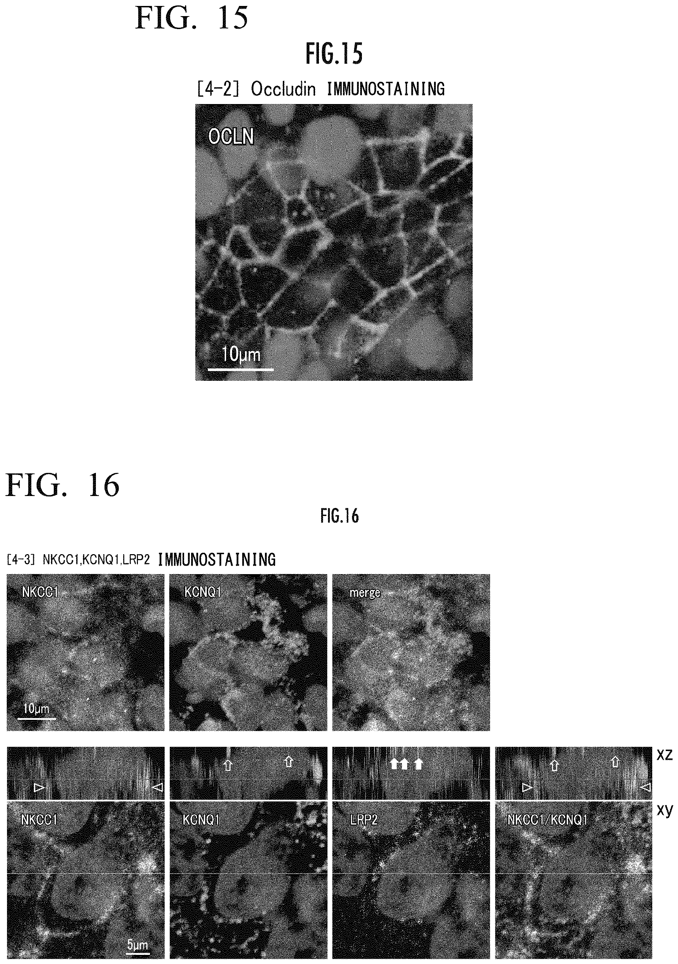

FIG. 15 is a diagram representing the results of, in Test Example 4, inducing differentiation from inner ear progenitor cells into inner ear stria vascularis marginal cells by co-culture with the feeder cells (melanocytes) that have been separately subjected to adhesion culture in advance, and detecting the expression of Occludin, which is a known intercellular tight junction protein in the inner ear stria vascularis marginal cells, by immunostaining with the specific antibody.
FIG. 16 is a diagram representing the results of, in Test Example 4, inducing differentiation from inner ear progenitor cells into inner ear stria vascularis marginal cells by co-culture with the feeder cells (melanocytes) that have been separately subjected to adhesion culture in advance, and detecting the expression of NKCC1, KCNQ1, and LRP2, which are known functional proteins in the inner ear stria vascularis marginal cells, by immunostaining with each of the specific antibodies.

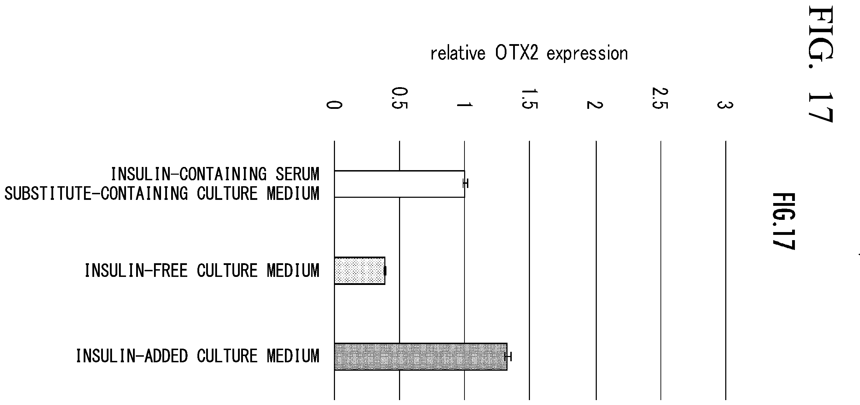

FIG. 17 is a diagram representing the results of quantitatively determining the gene expression of OTX2 by qPCR in a process of inducing differentiation from inner ear progenitor cells into inner ear stria vascularis marginal cells in Test Example 5, in a case where a culture medium containing an insulin-containing serum substitute, an insulin-free culture medium, or a culture medium obtained by adding insulin to an insulin-free culture medium is used as the culture medium.

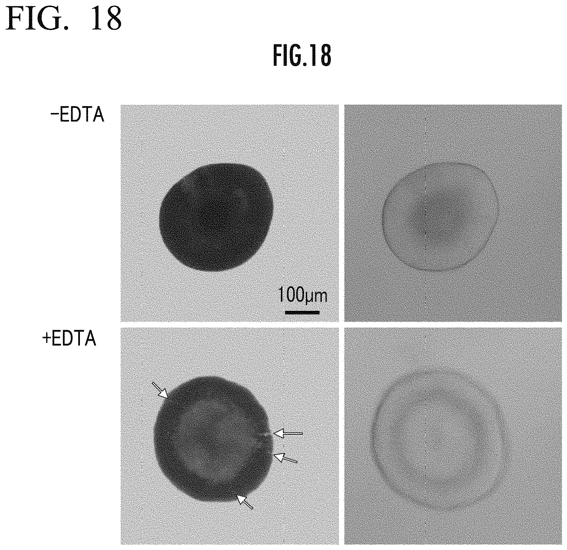

FIG. 18 is a diagram representing the results of performing a barrier function assay in Test Example 6, in which the left column represents photographs showing fluorescence microscopy images, and the right column is photographs showing bright field microscopy images.

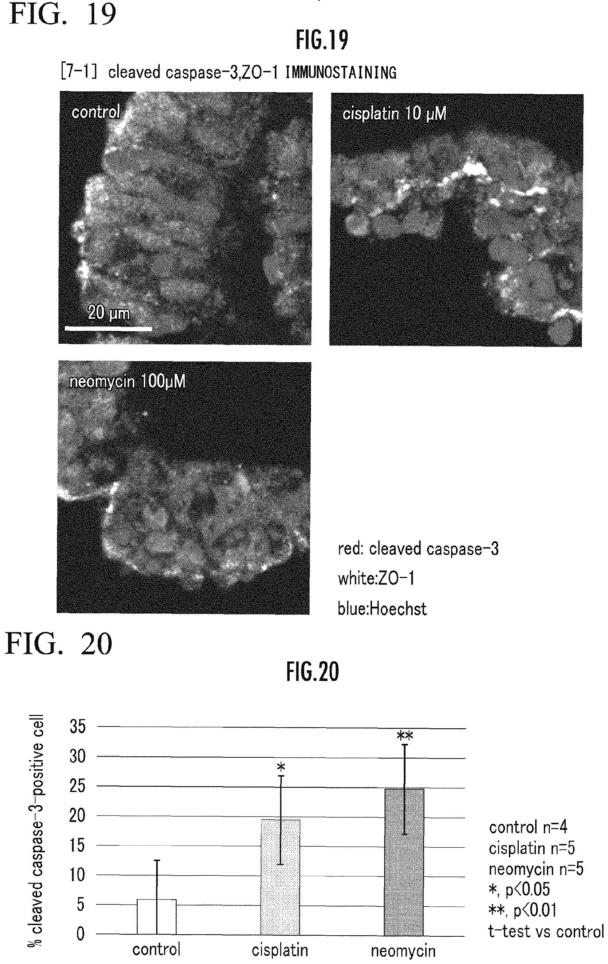

FIG. 19 is a diagram representing the results of, in Test Example 7, detecting the expression of ZO-1, which is a known intercellular tight junction protein, and cleaved caspase-3, which is a known apoptosis marker, by immunostaining with the specific antibody after agent treatment of the inner ear stria vascularis marginal cells obtained by inducing differentiation from pluripotent stem cells.
FIG. 20 is a diagram representing the results of, in Test Example 7, calculating the proportion of the cells in which cleaved caspase-3 is expressed, to the total number of cells after agent treatment of the inner ear stria vascularis marginal cells obtained by inducing differentiation from pluripotent stem cells.
FIG. 21 is an enlarged schematic explanatory diagram representing a stria vascularis portion in a cross section of the inner ear cochlea.

### [Description of Embodiments]

The present invention relates to a method for inducing differentiation into inner ear cells that constitute an inner ear organ, and more specifically, relates to a method for producing inner ear stria vascularis marginal cells, which has been improved to promote differentiation induction from stem cells such as pluripotent stem cells, and progenitor cells into inner ear stria vascularis marginal cells.

In the present specification, the term "pluripotent stem cell" has the same meaning as that generally understood by those skilled in the art, and refers to a stem cell having the ability to differentiate into almost all cells constituting the body. As pluripotent stem cells, an induced pluripotent stem cell (iPS cell), an embryonic stem cell (ES cell), and the like are known. In addition, the pluripotent stem cell may be derived from a human or may be derived from a non-human organism. Furthermore, a pluripotent stem cell which is prepared by reprogramming a somatic cell of a healthy person may be used, or a pluripotent stem cell which is prepared by reprogramming a somatic cell of a disease carrier may be used. The disease particularly includes diseases related to an inner ear organ, an auditory sense, auditory capacity, and the like, and examples thereof include Pendred syndrome, Usher syndrome, and the like.

In the present specification, the term "inner ear stria vascularis marginal cell" refers to a cell that exhibits the same physiological function as the stria vascularis marginal cell that is endogenous to the inner ear organ. However, the same physiological functionality as that of the stria vascularis marginal cells that are endogenous to the inner ear organ has a meaning that includes not only the case where all the original functionalities are provided, but also the case where some of the physiological functionalities are partially exhibited. Therefore, for example, it has a meaning that includes the inner ear stria vascularis marginal zone cell.

In the present specification, the term "adhesion culture" means culturing target cells or target cell population by allowing the cells or the cell population to adhere to a bottom surface or the like of a culture vessel. In addition, the term "suspension culture" means culturing target cells or target cell population without allowing the cells or the cell population to adhere to a bottom surface or the like of a culture vessel. In this case, the fact that the cells or the cell population adhere to the bottom surface of the culture vessel or the like during culturing means a state in which the cells or the cell population adhere to the bottom surface of the culture vessel or the like through cell-substrate adhesion molecules contained in an extracellular matrix (ECM) or the like, and refers to a state in which cells or cell population do not float in the culture solution even in a case where the culture solution is lightly shaken. On the other hand, in the culture, the fact that the cells or the cell population do not adhere to the bottom surface of the culture vessel or the like means a state in which the cells or the cell population are not adhered to the bottom surface of the culture vessel or the like through a cell-substrate adhesion molecule contained in an extracellular matrix (ECM) or the like, and refers to a state in which cells or cell population float in the culture solution by lightly shaking the culture solution even in a case where the cells or the cell population are in contact with the bottom surface of the culture vessel or the like. At the time of adhesion culture, in order to promote the adhesion of cells to the substrate, it is preferable that the bottom surface of the plastic dish be chemically treated or the bottom surface be coated with an adhesion coating agent (such as gelatin, polylysine, or agar) that promotes the adhesion. At the time of suspension culture, it is preferable that the surface of the bottom surface of the plastic dish or the like not be treated or be coated with an anti-adhesion coating agent (such as poly(2-hydroxyethyl methacrylate)) for preventing adhesion of cells to the substrate. In general, according to the adhesion culture, it is easy to perform an operation such as medium exchange for promoting differentiation induction or suppressing differentiation induction conversely, or regulating them. On the other hand, according to the suspension culture, three-dimensional organoid formation that mimics a tissue in a living body is easily promoted. Even in the case of the adhesion culture, it takes time for the target cells to adhere, and the cells may be in a floating state for a certain period of time. However, in a case where the cells finally adhere even though it takes time, they are included in the adhesion culture.

### [1] Differentiation induction from pluripotent stem cells into inner ear progenitor cells

The differentiation induction from pluripotent stem cells into inner ear progenitor cells can be achieved, for example, through the steps (1A) to (4A) as described below. However, the technical scope of the present invention is not limited to the use of the inner ear progenitor cells prepared by the method described below, and even the inner ear progenitor cells prepared by any other differentiation induction method can be used. For example, the inner ear progenitor cells used in the present invention can be prepared by the method described in Japanese Patent (Granted) Publication No. 6218152, the method described by Makoto Hosoya et al. (Cell Reports 18, 68-81, January 3, 2017), or the method described by Sho Kurihara et al. (Stem Cells Transl Med. 2022 Mar 31; 11 (3): 282-296.), or the like.

(1A) A step of culturing pluripotent stem cells in the absence of a growth factor and in the presence of a ROCK inhibitor
(2A) A step of culturing the cell population obtained in step (1A) in the absence of a growth factor and a ROCK inhibitor
(3A) A step of culturing the cell population obtained in step (2A) in the presence of at least one growth factor selected from the group consisting of bFGF, FGF3, FGF10, and FGF19 and in the presence of BMP4
(4A) A step of culturing the cell population obtained in step (3A) in the presence of at least one growth factor selected from the group consisting of bFGF, FGF3, FGF10, and FGF19, and in the absence of BMP4

The culture medium used in the above-described step (1A) may be any culture medium capable of maintaining pluripotent stem cells or cells that are directed to differentiation from pluripotent stem cells, and is not particularly limited. Preferred examples of the culture medium include "mTeSR1" (STEMCELL Technologies) or the like, which is a serum-free culture medium that does not require feeder cells for maintaining pluripotent stem cells. However, in step (1A), the culture is performed in the absence of a growth factor and in the presence of an inhibitor of Rho-associated coiled-coil forming kinase/Rho binding kinase (ROCK). The ROCK inhibitor has an effect of suppressing cell death on pluripotent stem cells. Step (1A) is preferably performed for 1 to 3 days, and more preferably performed for 1 to 2 days.

Examples of the ROCK inhibitor used in the above-described step (1A) include Y-27632 ((R)-(+)-trans-N-(4-Pyridyl)-4-(1-aminoethyl)-cyclohexanecarboxamide), Fasudil hydrochloride, K-115 (Ripasudil hydrochloride hydrate), DE-104, and the like. The concentration of the ROCK inhibitor may be determined to be an appropriate optimum concentration according to the type of the ROCK inhibitor, but for example, in a case of Y-27632, the concentration is preferably 1 to 100 µM and more preferably 10 to 20 µM.

The culture medium used in the above-described step (2A) may be any culture medium capable of maintaining pluripotent stem cells or cells that are directed to differentiation from pluripotent stem cells, and is not particularly limited. As well as the culture medium preferably used for the culture in step (1A), preferred examples of the culture medium include "mTeSR1" (STEMCELL Technologies) or the like, which is a serum-free culture medium that does not require feeder cells for maintaining pluripotent stem cells. However, in step (2A), the culture is performed in the absence of a growth factor and in the absence of a ROCK inhibitor. In addition, in that case, in a preferable aspect, it is preferable that the culture medium be exchanged with a basal culture medium (for example, a serum-free culture medium "DMEM/F12" (trade name "D-MEM/Ham's F-12", FUJIFILM Wako Pure Chemical Corporation) which is used in the subsequent step, after culturing in the above-described mTeSR1 medium for about 1 day, and then be cultured while exchanging with a fresh culture medium every day, to maintain the cells. According to this, the cells can be well adapted to the basal culture medium used to allow the growth factor to act. In addition, appropriately, a supplemental nutritional ingredient may be added to the basal culture medium. Examples of the supplemental nutritional ingredient include a serum-free supplement "B27" (trade name "Gibco B-27 Supplement", Thermo Fisher Scientific, Inc.), a serum-free supplement "N2" (trade name "Gibco N-2 Supplement" (Thermo Fisher Scientific, Inc.), a serum-free supplement "Gibco GlutaMAX" (Thermo Fisher Scientific, Inc.), and a serum-free supplement "Nonessential amino acid" (Nacalai Tesque, Inc.), and the like. Step (2A) is preferably performed for 1 to 10 days in total, and more preferably performed for 2 to 8 days. Step (2A) is still more preferably performed for 3 to 6 days.

In the above-described steps (1A) and (2A), the meaning of the absence of a growth factor and/or a ROCK inhibitor is that a growth factor and/or a ROCK inhibitor may be substantially absent, and a growth factor and/or a ROCK inhibitor may be contained at a concentration level at which there is no effect.

The culture medium used in the above-described step (3A) may be any culture medium capable of maintaining cells that are directed to differentiation from pluripotent stem cells, and is not particularly limited. As well as the culture medium preferably used for the latter half culture in step (2A), preferred examples of the culture medium include a serum-free culture medium "DMEM/F12" (trade name "D-MEM/Ham's F-12", FUJIFILM Wako Pure Chemical Corporation) supplemented with a serum-free supplement "B27" (trade name "Gibco B-27 Supplement", Thermo Fisher Scientific, Inc.), a serum-free supplement "N2" (trade name "Gibco N-2 Supplement" (Thermo Fisher Scientific, Inc.), a serum-free supplement "Gibco GlutaMAX" (Thermo Fisher Scientific, Inc.), and a serum-free supplement "Nonessential amino acid" (Nacalai Tesque, Inc.), and the like. However, in step (3A), the culture is performed in the presence of at least one growth factor selected from the group consisting of bFGF, FGF3, FGF10, and FGF19 as the growth factor, and in the presence of BMP4. At that time, it is preferable that bFGF, FGF3, FGF10, and FGF19 all be present. The concentration range of bFGF, FGF3, FGF10, FGF19, and BMP4 in the culture medium, which are growth factors, is preferably 10 to 50 ng/mL, for each of the growth factors, and more preferably 10 to 25 ng/mL. In addition, it is preferable to maintain the cells by culturing the cells in a culture medium while exchanging the culture medium with a fresh culture medium, for example, approximately every day. According to this, the effect of directing to the desired differentiation by the above-described growth factors can be further enhanced. Step (3A) is preferably performed for 1 to 6 days in total, and more preferably performed for 2 to 5 days. Step (3A) is still more preferably performed for 3 to 4 days.

The culture medium used in the above-described step (4A) may be any culture medium capable of maintaining cells that are directed to differentiation from pluripotent stem cells, and is not particularly limited. As well as the culture medium preferably used for the culture in step (3A), preferred examples of the culture medium include a serum-free culture medium "DMEM/F12" (trade name "D-MEM/Ham's F-12", FUJIFILM Wako Pure Chemical Corporation) supplemented with a serum-free supplement "B27" (trade name "Gibco B-27 Supplement", Thermo Fisher Scientific, Inc.), a serum-free supplement "N2" (trade name "Gibco N-2 Supplement" (Thermo Fisher Scientific, Inc.), a serum-free supplement "Gibco GlutaMAX" (Thermo Fisher Scientific, Inc.), and a serum-free supplement "Nonessential amino acid" (Nacalai Tesque, Inc.), and the like. However, in step (4A), the culture is performed in the presence of at least one growth factor selected from the group consisting of bFGF, FGF3, FGF10, and FGF19 as the growth factor, and in the absence of BMP4. At that time, it is preferable that bFGF, FGF3, FGF10, and FGF19 all be present. The concentration range of bFGF, FGF3, FGF10, and FGF19 in the culture medium, which are growth factors, is preferably 10 to 50 ng/mL for each of the growth factors, and more preferably 25 ng/mL. In addition, it is preferable to maintain the cells by culturing the cells in a culture medium while exchanging the culture medium with a fresh culture medium, for example, approximately every day. According to this, the effect of directing to the desired differentiation by the above-described growth factors can be further enhanced. Step (4A) is preferably performed for 1 to 6 days in total, and more preferably performed for 2 to 5 days. Step (4A) is still more preferably performed for 3 to 4 days.

In step (4A), the meaning of the absence of BMP4 is that BMP4 may be substantially absent, and may be contained at a concentration level at which there is no effect.

The series of cultures in steps (1A) to (4A) are preferably performed by adhesion culture in which the cells are cultured while being allowed to adhere to the bottom surface of the culture dish or the like. According to this, the cells can be efficiently cultured. In addition, it is easy to perform an operation such as medium exchange for promoting differentiation induction or suppressing differentiation induction conversely, or regulating them. Furthermore, it is preferable that the cells be cultured in a serum-free culture medium. According to this, the risk of differentiation into unintended cells caused by serum factor can be suppressed.

Here, in general, in order to assess the degree of differentiation into inner ear cells, the expression of PAX2 or PAX8, which is an inner ear precursor cell marker, is used as an index. That is, in a case where the progenitor cells in which the degree of differentiation is immature is directed to the differentiation into inner ear cells through a predetermined culture, the expression of PAX2 or PAX8 is increased (Reference 1: Ealy M, Ellwanger DC, Kosaric N, Stapper AP, Heller S, "Single-cell analysis delineates a trajectory toward the human early otic lineage." Proc Natl Acad Sci U S A. 2016 Jul 26, 113 (30): 8508-13; Reference 2: Koehler KR, Nie J, Longworth-Mills E, Liu XP, Lee J, Holt JR, Hashino E, "Generation of inner ear organoids containing functional hair cells from human pluripotent stem cells." Nat Biotechnol. 2017 Jun, 35 (6): 583-589). Therefore, the degree of differentiation into the inner ear cells can be assessed by examining the expression of PAX2 and PAX8 at the protein expression level or the mRNA expression level, also in the inner ear progenitor cells obtained through the method described above. That is, in a case where the expression of PAX2 or PAX8 is examined at a protein expression level or an mRNA expression level, the cell population has reached a level at which at least the expression can be detected. Here, as an index for assessing the degree of differentiation into inner ear cells, in addition to PAX2 and PAX8, FoxG1, GATA3, TFAP2A, ECAD, SOX10, JAG1, and the like are also well known markers for inner ear progenitor cells, and any one or more of the markers including these may be used for assessment. In the present specification, the term "inner ear precursor cell" refers to a cell before differentiation, which has an ability to differentiate into inner ear cells that constitute the inner ear organ. Therefore, it has a meaning that includes, for example, a cell referred to as an inner ear stem cell, an ectodermal placode cell, a cochlear stem cell, a cochlear precursor cell, a tissue stem cell contained in the tissue of the inner ear organ, or the like.

### [2] Differentiation induction from inner ear progenitor cells into inner ear stria vascularis marginal cells

FIG. 1 shows a flowchart representing one embodiment of a method for producing inner ear stria vascularis marginal cells according to the present invention. As shown in FIG. 1, in the present invention, a specific treatment is performed on inner ear progenitor cells to induce differentiation from inner ear progenitor cells into more mature inner ear cells. Specifically, the cell population including inner ear progenitor cells is cultured in an insulin-free culture medium that does not contain insulin or contains insulin only in a trace amount, to induce differentiation into inner ear stria vascularis marginal cells.

As the basal culture medium used for the culture in the insulin-free culture medium, for example, as well as the case of preparing the inner ear progenitor cells described above, "DMEM/F12" (trade name "D-MEM/Ham's F-12", FUJIFILM Wako Pure Chemical Corporation) or the like can be used. In addition, appropriately, a supplemental nutritional ingredient may be added to the basal culture medium. Examples of the supplemental nutritional ingredient include a serum-free supplement "Gibco GlutaMAX" (Thermo Fisher Scientific, Inc.), a serum-free supplement "Nonessential amino acid" (Nacalai Tesque, Inc.), and the like. However, the insulin-free culture medium used in the present invention needs to be limited in the insulin concentration in the culture medium. As shown in Examples described later, this is because the differentiation induction into the target marginal cells is hindered in a culture medium having a normal insulin concentration. The lower limit value of the insulin concentration is 0 nM or more, and the upper limit value thereof is preferably, for example, 100 nM or less. The upper limit value of the insulin concentration may be 90 nM or less, 80 nM or less, 70 nM or less, 60 nM or less, 50 nM or less, 40 nM or less, 30 nM or less, 20 nM or less, 10 nM or less, 5 nM or less, 4 nM or less, 3 nM or less, 2 nM or less, 1 nM or less, or the like.

In the preparation of the inner ear progenitor cells described above, as serum-free supplements that can be added to and replenished in the basal culture medium, a serum-free supplement "B27" (trade name "Gibco B-27 Supplement", Thermo Fisher Scientific, Inc.), a serum-free supplement "N2" (trade name "Gibco N-2 Supplement" (Thermo Fisher Scientific, Inc.), and the like are shown as examples, and these contain insulin at a concentration of a certain level or higher. Therefore, it is not preferable that the supplement be added to and replenished in the insulin-free culture medium described above in a normal amount according to these product protocols. On the other hand, as a serum-free supplement containing similar ingredients, there is a serum-free supplement "N21-Ins" (trade name "N21-MAX InsulinFree Media Supplement", R&D Systems, Inc.) containing no insulin. In a case of a culture supplement product that does not contain such insulin, it is possible to be added to and replenished in the above-described insulin-free culture medium used in the present invention in a normal amount according to the product protocol.

Here, generally, as growth factors that contribute to the differentiation induction from inner ear progenitor cells into more mature inner ear cells, EGF, bFGF, FGF3, BMP4, and the like are known. Therefore, in the culture with the above-described insulin-free culture medium, it is possible to perform the culture in the culture medium containing one or more of these growth factors. In this case, the concentration range of these growth factors in the culture medium is, for EGF, preferably 10 to 50 ng/mL and more preferably 20 to 30 ng/mL. The concentration of bFGF is preferably 10 to 50 ng/mL, and more preferably 10 to 30 ng/mL. The concentration of FGF3 is preferably 10 to 100 ng/mL, and more preferably 20 to 80 ng/mL. The concentration of BMP4 is preferably 10 to 50 ng/mL, and more preferably 20 to 30 ng/mL.

The culture in the insulin-free culture medium is particularly preferably performed in an environment in which as growth factors, at least EGF is present and at least one or more selected from the group consisting of bFGF, FGF3, and BMP4 are further present. In particular, in suspension culture, it is important to culture the cells in a culture medium in which EGF, FGF3, and BMP4 are further contained as a culture medium that is first subjected to culture medium exchange as an insulin-free culture medium. More specifically, in the suspension culture in the insulin-free culture medium, it is preferable to perform the culture in the presence of at least EGF as a growth factor, and furthermore, it is more preferable to perform the culture medium exchange at each timing to allow each growth factor to be present, such as that in the first half of the suspension culture period, when the culture in the presence of FGF3 and BMP4 in addition to EGF is performed, in the middle of the suspension culture period, when the culture in the presence of FGF3 in addition to EGF is performed, and in the latter half of the suspension culture period, when the culture in the presence of bFGF in addition to EGF is performed.

On the other hand, in the culture with the insulin-free culture medium, it is more preferable that the culture medium not contain IGF-1. According to this, it is possible to avoid the provision of an insulin-like signal by IGF-1.

By using the insulin-free culture medium and by the presence of these preferred growth factors or by their replacement, it is possible to more reliably induce differentiation into the inner ear stria vascularis marginal cells. As the culture method, adhesion culture may be performed or suspension culture may be performed, but from the viewpoint that it is easy to promote the formation of a three-dimensional organoid that mimics the tissue in the living body, the suspension culture is preferably performed. Furthermore, it is preferable that the cells be cultured in a serum-free culture medium. According to this, the risk of differentiation into unintended cells caused by serum factor can be suppressed. In addition, the culture period is preferably 50 to 70 days in total from the start of the differentiation induction of pluripotent stem cells, more preferably 55 to 65 days, and still more preferably 60 to 65 days. In addition, the culture period in the insulin-free culture medium is preferably 30 to 50 days in total, more preferably 35 to 45 days, and still more preferably 40 to 45 days.

FIG. 2 shows a flowchart representing another embodiment of a method for producing inner ear stria vascularis marginal cells according to the present invention. In this embodiment, the differentiation into the inner ear stria vascularis marginal cells is more reliably induced by subjecting the inner ear progenitor cells differentiation-induced from pluripotent stem cells to the treatments of the following steps (1B) and (2B).

(1B) A step of subjecting the cell population including the inner ear progenitor cells differentiation-induced from the pluripotent stem cells to cell detaching and dispersing treatment
(2B) A step of subjecting the cells or the cell population obtained in step (1B) to suspension culture in the insulin-free culture medium in the presence of an extracellular matrix material

Specifically, as shown in FIG. 2, in the present embodiment, first, a cell detaching and dispersing treatment is performed on a cell population including inner ear progenitor cells differentiation-induced from pluripotent stem cells. In the process of inducing differentiation into inner ear cells by culturing pluripotent stem cells, in a case where the grown cells in a state where the cells adhere to each other are separated individually and transferred to the next culture, the cells are dissociated into single cells or a small number of cell masses of 2 to 10, and the individual cells are directly exposed to the culture medium or the culture vessel and are likely to be affected. In this case, in a case where the cells are undifferentiated or the growth activity of the cells is weak, the cells cannot survive and die. As a result, cells that are unnecessary for differentiation into inner ear cells are eliminated, and the expression levels of, for example, PAX2 and PAX8 can be reliably maintained. The cell detaching and dispersing treatment may be any means capable of detaching adhered cells from a culture substrate and dispersing the cells into individual cells, and is not particularly limited. Examples thereof include an enzyme treatment with a trypsin-like enzyme preparation (trade name "TrypLE Select", Thermo Fisher Scientific, Inc.), accutase (an enzyme preparation for cell detaching: trade name "Accutase", Nacalai Tesque, Inc.), or the like. After the enzyme treatment, the cells can be reliably dissociated from each other by pipetting or the like in the liquid culture medium. In addition, the remaining cell mass may be removed by passing through a mesh having a predetermined pore size. As a mesh used for such a purpose, a cell strainer provided with a nylon mesh having a predetermined pore size in a range of pore size of 1 to 1,000 µm or the like is commercially available. Therefore, a cell strainer having an appropriate pore size may be appropriately selected from such commercially available cell strainers and used.

In addition, as shown in FIG. 2, in the embodiment, the cells or cell population which have been subjected to cell detaching and dispersing treatment are subjected to suspension culture in an insulin-free culture medium that does not contain insulin or contains insulin only in a trace amount, in the presence of the extracellular matrix material. For suspension culture, it is preferable that cells or a cell population which have been subjected to cell detaching and dispersing treatment be suspended in an appropriate liquid culture medium and then cultured in a culture vessel for suspension culture capable of culturing in a non-adhesion state. As the culture vessel for non-adhesion culture, specifically, for example, a plastic dish for non-adhesion cell culture or the like can be used.

As the culture medium for the suspension culture, the insulin-free culture medium described above may be used. However, an extracellular matrix material is added thereto. The presence of the extracellular matrix material makes it easy to form the polarity of the cells and has an effect of serving as a scaffold for differentiation into stria vascularis marginal cells. The extracellular matrix material may be a material that functions as a scaffold in a case where cells grow three-dimensionally, and is not particularly limited, and examples thereof include Matrigel, Pronectin, collagen, laminin, and fibronectin. One kind of these may be used alone and two or more kinds thereof may be used in combination. The content of the extracellular matrix material in the insulin-free culture medium may be any range used by those skilled in the art, and is not particularly limited, but is typically, for example, in terms of protein content which is not limited, such as 0.01 to 10 mg/mL, may be 0.05 to 5 mg/mL, and may be 0.1 to 1 mg/mL.

This suspension culture is preferably performed, in total, for 30 to 50 days, more preferably for 35 to 45 days, and still more preferably for 40 to 45 days from the start of the suspension culture in the insulin-free culture medium. In addition, in the suspension culture, the suspension culture may be further continued by collecting the spheres (cell masses) formed by centrifugation or the like without breaking the spheres, and by replacing with the fresh culture medium or adding the fresh culture medium. In this case, it is preferable to perform the culture medium exchange and the addition of a fresh culture medium in the additional suspension culture every 3 to 4 days.

The three-dimensional cultured stria vascularis marginal cells that can be prepared in this way are cells that exhibit the same inherent physiological functionality as the stria vascularis marginal cells that are endogenous to the inner ear organ, such as the expression of intercellular tight junction proteins and ion transporters.

### [3] Two-dimensional culture of inner ear stria vascularis marginal cells

FIG. 3 shows a flowchart representing still another embodiment of a method for producing inner ear stria vascularis marginal cells according to the present invention. In the present embodiment, by treating the inner ear progenitor cells differentiation-induced from pluripotent stem cells with the following steps (1C) to (3C), the differentiation into the inner ear stria vascularis marginal cells is more reliably induced, and a two-dimensional structure (layered) is formed by the inner ear stria vascularis marginal cells. That is, it is possible to perform two-dimensional culture (2D culture) of the inner ear stria vascularis marginal cells.

(1C) A step of subjecting the cell population containing the inner ear progenitor cells differentiation-induced from the pluripotent stem cells to cell detaching and dispersing treatment
(2C) A step of subjecting the cells or the cell population obtained in step (1C) to suspension culture in the insulin-free culture medium in the presence of an extracellular matrix material
(3C) A step of seeding the cells or the cell population after the suspension culture in step (2C) on feeder cells that have been separately subjected to adhesion culture in advance, and culturing the cells or the cell population in the insulin-free culture medium

Specifically, as shown in FIG. 3, in the present embodiment, the steps (1C) and (2C) for suspension culture in the insulin-free culture medium are common to the steps (1B) and (2B) described in FIG. 2, but in the process, the cells of the cell population in suspension culture are appropriately collected, subjected to dispersing treatment by pipetting in a solution to which an enzyme is added as necessary, and seeded on feeder cells that have been separately subjected to adhesion culture in advance. Then, the culture in the insulin-free culture medium described above is further continued in that state. As the feeder cells, melanocytes, melanocyte-like cells, or the like can be used. As a result, the cells are seeded on the feeder cells formed in a two-dimensional structured shape (layered) along the surface of the two-dimensional structure such as the inner bottom surface of the culture container by subjecting feeder cells such as melanocytes to adhesion culture, and co-culture with the feeder cells is performed, thereby the formation of a two-dimensional structure (layered) is also promoted in the inner ear stria vascularis marginal cells.

The co-culture with the feeder cells is preferably performed for 30 to 50 days, more preferably for 35 to 45 days, and still more preferably for 40 to 45 days in total from the start. In addition, in the co-culture with the feeder cells, the co-culture with the feeder cells may be further continued by replacing with the fresh culture medium or adding the fresh culture medium. In this case, it is preferable to perform the culture medium exchange and the addition of a fresh culture medium in the additional co-culture every 3 to 4 days.

The two-dimensionally cultured inner ear stria vascularis marginal cells that can be prepared in this way are, as shown in Examples described later, the layered structure with a barrier function, and thus, can be more preferably used as a functional tool cell in which cells that exhibit the same inherent physiological functionality as the stria vascularis marginal cells that are endogenous to the inner ear organ, such as an intercellular tight junction function and unidirectional ion transport, which are required to maintain the homeostasis of the potassium ion concentration, have a layered structure and are planarly cultured.

### [4] Agent assessment method

From another viewpoint of the present invention, the present invention can provide an agent assessment method. That is, an agent assessment method is provided including a step of treating inner ear stria vascularis marginal cells differentiation-induced from pluripotent stem cells with a test agent, and a step of assessing a state of the inner ear stria vascularis marginal cells treated with the test agent.

In the agent assessment method according to the present invention, it is possible to cause any test agent to act on the inner ear stria vascularis marginal cells differentiation-induced from pluripotent stem cells and to assess what kind of influence the test agent has on the inner ear stria vascularis marginal cells. Therefore, the agent assessment method is useful, for example, as a tool for effectively and efficiently screening a substance related to the functionality of the inner ear organ. The step of treating with the test agent is not limited, but may be performed, for example, such that the inner ear stria vascularis marginal cells obtained as described above are suspended in a buffer solution, the test agent is added to the solution at a predetermined concentration, and the state of the cells is observed after a predetermined time has elapsed, or such that in the culture of the inner ear stria vascularis marginal cells obtained as described above, the test agent at a predetermined concentration is added to the culture medium for the culture, the culture is performed for a predetermined time, and then the state of the cells is observed. In addition, the step of assessing the state of the inner ear stria vascularis marginal cells after the treatment is not limited, but for example, ion permeability, barrier function, apoptosis, oxidative stress, and the like of the cells related to the functionality of the inner ear organ may be examined.

### [5] Cell culture for assessing an agent

From still another viewpoint of the present invention, the present invention provides a cell culture for assessing an agent. That is, a cell culture for assessing an agent is provided, which contains inner ear stria vascularis marginal cells differentiation-induced from pluripotent stem cells.

According to the cell culture for assessing an agent of the present invention, it is possible to cause any test agent to act on the cell culture and to assess what kind of influence the test agent has on the inner ear stria vascularis marginal cells. Therefore, the cell culture is useful, for example, as a tool for effectively and efficiently screening a substance related to the functionality of the inner ear organ. The form of the cell culture may be any form of cells or a cell population including at least the above-mentioned inner ear stria vascularis marginal cells, and is usually a form of a cell mass obtained by suspension culture. In this case, the cell culture is preferably preserved in a preservation solution such as a medium or a culture medium for the purpose of protecting or storing the form of a cell mass. In a case of using the cell culture, the preservation solution is replaced with a test solution containing the test agent, and the state of the cells is observed after a predetermined time has elapsed, thereby the cell culture can be used for assessing a test agent.

Alternatively, since the cells can also be formed in a two-dimensionally layered structure (layered shape) along the surface of the two-dimensional structure such as the inner bottom surface of the culture container by the co-culture with feeder cells such as melanocytes as described above, as the form of the cell culture, a form in which the cells are two-dimensionally cultured (2D culture) in this manner can be adopted. In this case, the cell culture is preferably preserved in the culture container with the preservation solution such that the cell culture is covered with the preservation solution such as a medium or a culture medium for the purpose of protecting or storing the form of the two-dimensional structured shape (layered). In a case of using the cell culture, the preservation solution is replaced with a test solution containing the test agent, and the state of the cells is observed after a predetermined time has elapsed, thereby the cell culture can be used for assessing a test agent. The assessment is not limited, but for example, ion permeability, barrier function, apoptosis, oxidative stress, and the like of the cells related to the functionality of the inner ear organ may be examined.

### [Examples]

Hereinafter, the present invention will be described more specifically with reference to examples. However, these examples do not limit the scope of the present invention.

### (Reagent)

(1) Matrigel: a coating agent (trade name "Corning Matrigel Basement Membrane Matrix", Corning Inc.)
(2) Accutase: an enzyme preparation for cell detaching (trade name "Accutase", Nacalai Tesque, Inc.)
(3) Trypsin-like enzyme preparation (trade name "TrypLE Select", Thermo Fisher Scientific, Inc.)
(4) Y-27632: a ROCK inhibitor (specific inhibitor of Rho-associated coiled-coil forming kinase/Rho binding kinase) (trade name "Y-27632", FUJIFILM Wako Pure Chemical Corporation)
(5) mTeSR1: a maintenance culture medium for iPS cells (trade name "mTeSR1", STEMCELL Technologies)
(6) DMEM/F12: a serum-free culture medium (trade name "D-MEM/Ham's F-12", FUJIFILM Wako Pure Chemical Corporation)
(7) B27: a serum-free supplement (50X) (trade name "Gibco B-27 Supplement", Thermo Fisher Scientific, Inc.)
(8) N2: a serum-free supplement (100X) (trade name "Gibco N-2 Supplement", Thermo Fisher Scientific, Inc.)
(9) N21-Ins: a serum-free supplement (50X) (trade name "N21-MAX InsulinFree Media Supplement", R&D Systems, Inc.)
(10) GlutaMAX: a serum-free supplement (100X) (trade name "Gibco GlutaMAX", Thermo Fisher Scientific, Inc.)
(11) Nonessential amino acid (100X): a nonessential amino acid supplement (trade name "MEM Non-essential Amino Acids Solution", Nacalai Tesque, Inc.)
(12) Poly-L-ornithine/fibronectin: a coating agent (trade name "Poly-L-ornithine", Sigma-Aldrich Co. LLC), (trade name "Fibronectin", Sigma-Aldrich Co. LLC)
(13) bFGF (trade name "Recombinant Human FGF-basic", Peprotech, Inc.)
(14) FGF3 (trade name "Recombinant Human FGF-3 protein", R&D Systems, Inc.)
(15) FGF10 (trade name "Recombinant Human FGF-10 protein", Peprotech, Inc.)
(16) FGF19 (trade name "Recombinant Human FGF-19 protein", Peprotech, Inc.)
(17) BMP4 (trade name "Recombinant Human BMP-4 protein", Peprotech, Inc.)
(18) 1GF-1 (trade name "Recombinant Human 1GF-1 Protein", R&D Systems, Inc.)
(19) SB431542: a TGF-β receptor inhibitor (trade name "SB431542", ReproCELL Incorporated)
(20) Heparin (trade name "Heparan sulfate sodium salt", Sigma-Aldrich Co. LLC)
(21) L-glutamine (trade name "L-Glutamine", Nacalai Tesque, Inc.)
(22) iMatrix-511 silk: a coating agent (trade name "iMatrix-511 silk", nippi, Incorporated)
(23) StemFit AK02N: a maintenance culture medium for iPS cells (trade name "StemFit AK02N", Ajinomoto Co., Inc.)
(24) CHIR99021: a GSK-3 inhibitor (trade name "CHIR-99021", Focus biomolecules)
(25) The sequences of the primers used for qPCR are shown in Table 1.

**[Table 1]**

| [PCR primer] | | | |
|---|---|---|---|
| Transcript | Direction | Sequence | SEQ ID NO. |
| PAX2 | Forward | TCAAGTCGAGTCTATCTGCATCC | SEQ ID NO. 1 |
| | Reverse | CATGTCACGACCAGTCACAAC | SEQ ID NO. 2 |
| PAX8 | Forward | GCCCAGTGTCAGCTCCATTA | SEQ ID NO. 3 |
| | Reverse | GCTGTCCATAGGGAGGTTGAA | SEQ ID NO. 4 |
| OTX2 | Forward | CAAAGTGAGACCTGCCAAAAAGA | SEQ ID NO. 5 |
| | Reverse | TGGACAAGGGATCTGACAGTG | SEQ ID NO. 6 |
| ACTB | Forward | TGAAGTGTGACGTGGACATC | SEQ ID NO. 7 |
| | Reverse | GGAGGAGCAATGATCTTGAT | SEQ ID NO. 8 |

In the culture medium for cell culture of the following test examples, ampicillin was appropriately used as an antibacterial agent as necessary in a concentration of 100 µg/mL. In addition, in a case where it is not particularly mentioned, the culture was usually performed under normal oxygen conditions (20% of O₂, 5% of CO₂).

### [Test Example 1]

In the present test example, the differentiation from human iPS cells into inner ear progenitor cells was induced.

### [Differentiation induction method]

### (Before differentiation induction: (-)Day 2)

1) A 6-well plate was coated with Matrigel.
2) Accutase was added to confluent feeder-free human iPS cells, and the cells were incubated at 37°C for 2 to 3 minutes and detached off from the dish.
3) The cells were diluted with PBS and then centrifuged to collect the cells.
4) The supernatant was removed, and the cells were suspended in an mTeSR1 medium to which a ROCK inhibitor (Y-27632) (10 µM) was added.
5) The number of cells was counted with a hemocytometer after passing through a nylon mesh (pore size: 40 µm).
6) The mTeSR1 medium to which Y-27632 was added was added to the well coated with Matrigel in 1).
7) The cell suspension was seeded to be 2.5 × 10⁴ cells/cm² per well.

### (Before differentiation induction: (-)Day 1)

The culture medium was exchanged to an mTeSR1 medium not containing a ROCK inhibitor.

### (Day 0)

The culture medium was exchanged with a serum-free culture medium (DMEM/F12 + 2% B27 + 1% N2 + 1% GlutaMAX + 1% Nonessential amino acid). Thereafter, the culture medium was exchanged every day until Day 2.

### (Day 3)

The culture medium was exchanged with a culture medium obtained by adding growth factors bFGF, FGF3, FGF10, FGF19, and BMP4 to a serum-free culture medium (DMEM/F12 + 2% B27 + 1% N2 + 1% GlutaMAX + 1% Nonessential amino acid) such that the concentrations of the growth factors were each 25 ng/mL, 25 ng/mL, 25 ng/mL, 25 ng/mL, and 10 ng/mL. Thereafter, the culture medium was exchanged every day until Day 5.

### (Day 6)

The culture medium was exchanged with a culture medium obtained by adding growth factors bFGF, FGF3, FGF10, and FGF19 to a serum-free culture medium (DMEM/F12 + 2% B27 + 1% N2 + 1% GlutaMAX + 1% Nonessential amino acid) (concentrations of all growth factor were 25 ng/mL), and the culture was performed until Day 8.

### (Day 9)

Accutase was added to the cells, and the cells were incubated at 37°C for 2 to 3 minutes, detached off from the dish, and diluted with PBS. The cells were collected by centrifugation, and L-glutamine was added to a serum-free culture medium (DMEM/F12 + 2% B27 + 1 % N2) to a concentration of 2 mM. Furthermore, the cells were suspended in the culture medium prepared by adding the growth factors bFGF, FGF3, FGF10, and FGF19 such that the concentration of each growth factor was 25 ng/mL. This cell suspension was seeded in wells coated with poly-L-ornithine/fibronectin such that the cell concentration was approximately 1/3 of the cell concentration before the cell detaching treatment per well, and adhesion culture was performed under low oxygen conditions (4% of O₂, 5% of CO₂).

### (Day 10)

The culture medium was exchanged with a culture medium prepared by adding L-glutamine to a serum-free culture medium (DMEM/F12 + 2% B27 + 1% N2) such that the concentration thereof was 2 mM, and further adding growth factors bFGF, EGF, and 1GF-1 thereto such that the concentrations of the growth factors were each 20 ng/mL, 20 ng/mL, and 50 ng/mL.

### (Day 12)

The total RNA was extracted from the cells on the 12th day (Day 12) from the start of differentiation induction using an RNeasy spin column kit (QIAGEN N.V.), cDNA was synthesized from 1 µg of total RNA of each sample using a reverse transcriptase (trade name "Invitrogen SuperScript IV Reverse Transcriptase (Thermo Fisher Scientific, Inc.), and the expression level of the marker gene was quantified by qPCR.

As a result, the expression of genes specific to the inner ear progenitor cells, such as PAX2 and PAX8, which are known marker molecules of the inner ear progenitor cells, was confirmed.

In addition, the expression state of various marker proteins in the cells on the 12th day (Day 12) from the start of differentiation induction was examined by immunostaining.

### [1] PAX2, PAX8, SOX2 (Day 12)

The cells on the 12th day (Day 12) from the start of differentiation induction were treated with 4% paraformaldehyde at room temperature for 20 minutes to be fixed, treated with 0.3% PBST for 30 minutes, and then subjected to blocking with 10% normal donkey serum/0.3% PBST at room temperature for 1 hour. As the primary antibodies, a mouse anti-PAX2 antibody, a rabbit anti-PAX8 antibody, and a goat anti-SOX2 antibody were each used at concentrations of 1:500, 1:500, and 1:500, and the reaction was performed at room temperature for 2 hours. The reactant was reacted with a fluorescently labeled secondary antibody specific to the IgG animal species of each of the primary antibodies at room temperature for 1 hour. In addition, the nucleus was stained with Hoechst 33258. FIG. 4 shows a microscopic observation image obtained with a confocal microscope (scale bar: 50 µm).

As a result, as shown in FIG. 4, the expression of PAX2, PAX8, and SOX2, known marker proteins of inner ear progenitor cells, was confirmed. Therefore, it was confirmed that the differentiation from the human iPS cells into the inner ear progenitor cells was induced by the above-described method.

### [Test Example 2]

In the present test example, the differentiation from inner ear progenitor cells into inner ear stria vascularis marginal cells was induced.

### [Differentiation induction method]

### (Day 12)

The differentiation from human iPS cells into inner ear progenitor cells was induced by the same method as in Test Example 1, the cells on the 12th day (Day 12) from the start of the differentiation induction were detached off with Accutase, an equal amount of PBS was added thereto, the cells were passed through a nylon mesh (pore size: 40 µm), and the number of cells was counted with a hemocytometer.

The cells were suspended to a concentration of 5 × 10⁵ cells/10 mL and seeded in a low adhesion 6-well plate (trade name "Corning Ultra-Low Attachment Plate", Corning Inc.), and suspension culture was started under low oxygen conditions (4% of O₂, 5% of CO₂). At this time, the cells were suspended and suspension culture was started using a culture medium prepared by adding growth factors bFGF, EGF, 1GF-1, FGF3, and FGF10 to a serum-free culture medium (DMEM/F12 + 2% B27 + 1% N2) such that the concentrations of the growth factors were each 10 ng/mL, 10 ng/mL, 25 ng/mL, 50 ng/mL, and 50 ng/mL, further adding L-glutamine such that the concentration was 2 mM, adding Y-27632 such that the concentration was 10 µM, adding Matrigel such that the concentration was 1 %, adding SB431542 such that the concentration was 2 µM, and adding heparin such that the concentration was 50 ng/mL.

### (Day 16)

On the 4th day (Day 16) after the start of the suspension culture, the total amount of the culture medium was exchanged with a culture medium having a composition in which Matrigel and Y-27632 were excluded from the culture medium at the time of the start of the suspension culture, and the suspension culture was further continued under low oxygen conditions (4% of O₂, 5% of CO₂).

### (Day 20)

On the 8th day (Day 20) after the start of the suspension culture, the total amount of the culture medium was exchanged using a culture medium prepared by adding growth factors EGF, FGF3, and BMP4 to a serum-free culture medium (DMEM/F12 + 2% N21-Ins) such that the concentrations of the growth factors were each 10 ng/mL, 50 ng/mL, and 10 ng/mL, further adding L-glutamine such that the concentration was 2 mM, and adding heparin such that the concentration was 50 ng/mL. Furthermore, the suspension culture was further continued under normal oxygen conditions (20% of O₂, 5% of CO₂).

### (Day 24)

On the 12th day (Day 24) after the start of the suspension culture, the total amount of the culture medium was exchanged using a culture medium prepared by adding growth factors EGF and FGF3 to a serum-free culture medium (DMEM/F12 + 2% N21-Ins) such that the concentrations of the growth factors were 10 ng/mL and 50 ng/mL, further adding L-glutamine such that the concentration was 2 mM, and adding SB431542 such that the concentration was 2 µM. Furthermore, the suspension culture was further continued under normal oxygen conditions (20% of O₂, 5% of CO₂).

### (Day 32)

On the 20th day (Day 32) after the start of the suspension culture, the total amount of the culture medium was exchanged using a culture medium prepared by adding growth factors bFGF and EGF to a serum-free culture medium (DMEM/F12 + 2% N21-Ins) such that the concentrations of the growth factors were each 2 ng/mL and 10 ng/mL, further adding L-glutamine such that the concentration was 2 mM, and adding SB431542 such that the concentration was 2 µM. Furthermore, the suspension culture was further continued under normal oxygen conditions (20% of O₂, 5% of CO₂).

### (Day 36)

On the 24th day (Day 36) after the start of the suspension culture, the total amount of the culture medium was exchanged using the culture medium having the same composition as that used for the culture medium exchange on Day 32, and the suspension culture was further continued under normal oxygen conditions (20% of O₂, 5% of CO₂).

### (Day 40)

On the 28th day (Day 40) after the start of the suspension culture, the total amount of the culture medium was exchanged using a culture medium prepared by adding a growth factor EGF to a serum-free culture medium (DMEM/F12 + 2% N21-Ins) such that the concentration thereof was 10 ng/mL, further adding L-glutamine such that the concentration was 2 mM, and adding SB431542 such that the concentration was 2 µM. Thereafter, the culture medium was exchanged once every 3 days until Day 63, and the suspension culture was further continued under normal oxygen conditions (20% of O₂, 5% of CO₂).

The expression state of various marker proteins in the cells in the process of differentiation induction by the above-described method and in the cells after the differentiation induction was examined by immunostaining.

### [2-1] KCNQ1, KCNE1, LRP2 (Day 63)

The cells on the 51st day (Day 63) after the start of the suspension culture were treated with 4% paraformaldehyde at 4°C for 3 hours to be fixed, and a 7 µm frozen section was prepared. The frozen section was treated with 0.3% PBST for 10 minutes and then subjected to blocking with 10% normal donkey serum/0.1% PBST at room temperature for 1 hour. As the primary antibodies, a mouse anti-LRP antibody, a rabbit anti-KCNE1 antibody, and a goat anti-KCNQ1 antibody were each used at concentrations of 1:200, 1:200, and 1:100, and reacted at 4°C overnight. The reactant was reacted with a fluorescently labeled secondary antibody specific to the IgG animal species of each of the primary antibodies at room temperature for 1 hour. In addition, the nucleus was stained with Hoechst 33258. FIG. 5 shows microscopic observation images obtained with a confocal microscope (scale bar: 20 µm in the left column, 20 µm in the right column).

As a result, as shown in FIG. 5, the expression of KCNQ1, KCNE1, and LRP2, which are known functional proteins in the inner ear stria vascularis marginal cells, was detected. In addition, it was considered that they are co-localized on the cell membrane and can reproduce the expression pattern in the endogenous marginal cells.

### [2-2] NKCC1, KCNQ1 (Day 63)

The cells on the 51st day (Day 63) after the start of the suspension culture were treated with 4% paraformaldehyde at 4°C for 3 hours to be fixed, and a 7 µm frozen section was prepared. The frozen section was treated with 0.3% PBST for 10 minutes and then subjected to blocking with 10% normal donkey serum/0.1% PBST at room temperature for 1 hour. As the primary antibodies, a rabbit anti-KCNQ1 antibody and a goat anti-NKCC1 antibody were used at a concentration of 1:200 each, and reacted at 4°C overnight. The reactant was reacted with a fluorescently labeled secondary antibody specific to the IgG animal species of each of the primary antibodies at room temperature for 1 hour. In addition, the nucleus was stained with Hoechst 33258.

FIG. 6 shows a microscopic observation image obtained with a confocal microscope (scale bar: 50 µm).

As a result, as shown in FIG. 6, the expression of NKCC1 and KCNQ1, which are known functional proteins in the inner ear stria vascularis marginal cells, was detected. In addition, it was considered that they are not co-localized on the cell membrane and can reproduce the expression pattern in the endogenous marginal cells.

### [2-3] Na/K ATPase (Day 24)

The cells on the 12th day (Day 24) after the start of the suspension culture were treated with 10% TCA at 4°C for 15 minutes to be fixed, and blocking was performed at room temperature for 1 hour with 10% normal donkey serum/0.1% PBST. As the primary antibody, a rabbit anti-Na/K ATPase antibody was used at a concentration of 1:100 and reacted at 4°C overnight. The primary antibody was reacted with a fluorescently labeled secondary antibody specific to the IgG animal species at room temperature for 1 hour and observed with a confocal microscope. FIG. 7 shows a microscopic observation image obtained with a confocal microscope (scale bar: 20 µm).

As a result, as shown in FIG. 7, the expression of Na/K ATPase, a known functional protein in the inner ear stria vascularis marginal cells, was detected. In addition, it was considered that it is localized on the cell membrane and can reproduce the expression pattern in the endogenous marginal cells.

### [2-4] LMX1, ESRRB (Day 40)

The cells on the 28th day (Day 40) from the start of suspension culture were treated with 4% paraformaldehyde at room temperature for 20 minutes to be fixed, treated with 0.3% PBST for 20 minutes, and then subjected to blocking with 10% normal donkey serum/0.3% PBST at room temperature for 1 hour. As the primary antibodies, a mouse anti-ESRRB antibody and a rabbit anti-LMX1 antibody were used at a concentration of 1:100 each and reacted at 4°C overnight. The reactant was reacted with a fluorescently labeled secondary antibody specific to the IgG animal species of each of the primary antibodies at room temperature for 2 hours. In addition, the nucleus was stained with Hoechst 33258. FIG. 8 shows a microscopic observation image obtained with a confocal microscope (scale bar: 20 µm).

As a result, as shown in FIG. 8, the expression of LMX1 and ESRRB, which are known functional proteins in the inner ear stria vascularis marginal cells, was detected. In addition, it was considered that they are localized on the nucleus and can reproduce the expression pattern in the endogenous marginal cells.

### [2-5] Occludin (Day 52)

The cells on the 40th day (Day 52) from the start of suspension culture were treated with 10% TCA at 4°C for 15 minutes to be fixed, treated with 0.3% PBST for 10 minutes, and then subjected to blocking with 10% normal donkey serum/0.1% PBST at room temperature for 1 hour. As the primary antibody, a rabbit anti-Occludin antibody was used at a concentration of 1:100 and reacted at 4°C overnight. The primary antibody was reacted with a fluorescently labeled secondary antibody specific to the IgG animal species at room temperature for 1 hour and observed with a confocal microscope. FIG. 9 shows a microscopic observation image obtained with a confocal microscope (scale bar: 20 µm).

As a result, as shown in FIG. 9, the expression of Occludin, a known intercellular tight junction protein in the inner ear stria vascularis marginal cells, was detected. In addition, a characteristic cobblestone-like signal was shown, and it was considered that the differentiation-induced cells were inner ear stria vascularis marginal cells which were epithelial cell-like cells.

### [2-6] ZO-1 (Day 43)

The cells on the 31st day (Day 43) after the start of the suspension culture were treated with 10% TCA at 4°C for 15 minutes to be fixed, and blocking was performed at room temperature for 1 hour with 10% normal donkey serum/0.1% PBST. As the primary antibody, a goat anti-ZO-1 antibody was used at a concentration of 1:100 and reacted at 4°C overnight. The primary antibody was reacted with a fluorescently labeled secondary antibody specific to the IgG animal species at room temperature for 2 hours and observed with a confocal microscope. FIG. 10 shows a microscopic observation image obtained with a confocal microscope (scale bar: 20 µm).

As a result, as shown in FIG. 10, the expression of ZO-1, a known functional protein in the inner ear stria vascularis marginal cells, was detected. In addition, a cobblestone-like signal characteristic of the epithelial cell was shown, and it was considered that the differentiation-induced cells were stria vascularis marginal cells which were epithelial cell-like cells.

From the above results, it was clarified that, by culturing using the present induction method, the inner ear progenitor cells obtained by inducing differentiation from pluripotent stem cells can be further differentiation-induced into inner ear stria vascularis marginal cells.

### [Test Example 3]

In the present test example, the differentiation from human iPS cells into inner ear progenitor cells was induced by a method different from that in Test Example 1, and the differentiation from the inner ear progenitor cells into inner ear stria vascularis marginal cells was induced.

### [Differentiation induction method of inner ear progenitor cells]

### (Before differentiation induction: (-)Day 2)

1) A 6-well plate was coated with iMatrix-511 silk.
2) Accutase was added to confluent feeder-free human iPS cells, and the cells were incubated at 37°C for 2 to 3 minutes and detached off from the dish.
3) The cells were diluted with PBS and then centrifuged to collect the cells.
4) The supernatant was removed, and the cells were suspended in a StemFit AK02N to which a ROCK inhibitor (Y-27632) (10 µM) was added.
5) The number of cells was counted with a hemocytometer after passing through a nylon mesh (pore size: 40 µm).
6) The StemFit AK02N to which Y-27632 was added was added to the well coated with iMatrix-511 silk in 1).
7) The cell suspension was seeded to be 2.5 × 10⁴ cells/cm² per well.

### (Before differentiation induction: (-)Day 1)

The culture medium was exchanged with StemFit AK02N not containing a ROCK inhibitor.

### (Day 0)

The culture medium was exchanged with a serum-free culture medium (DMEM/F12 + 2% B27 + 1% N2 + 1% GlutaMAX + 1% Nonessential amino acid). Thereafter, the culture medium was exchanged every day until Day 2.

### (Day 3)

The culture medium was exchanged with a culture medium obtained by adding growth factors bFGF, FGF3, FGF10, FGF19, and BMP4 to a serum-free culture medium (DMEM/F12 + 2% B27 + 1% N2 + 1% GlutaMAX + 1% Nonessential amino acid) such that the concentrations of the growth factors were each 25 ng/mL, 25 ng/mL, 25 ng/mL, 25 ng/mL, and 10 ng/mL. Thereafter, the culture medium was exchanged every day until Day 5.

### (Day 6)

The culture medium was exchanged with a culture medium obtained by adding growth factors bFGF, FGF3, FGF10, FGF19, CHIR99021 to a serum-free culture medium (DMEM/F12 + 2% B27 + 1 % N2 + 1 % GlutaMAX + 1% Nonessential amino acid) (concentrations of all growth factor were 25 ng/mL, concentration of CHIR99021 as a GSK-3 inhibitor was 8 µM), and the culture was performed until Day 8.

### (Day 9)

Accutase was added to the cells, and the cells were incubated at 37°C for 2 to 3 minutes, detached off from the dish, and diluted with PBS. The cells were collected by centrifugation, and L-glutamine was added to a serum-free culture medium (DMEM/F12 + 2% B27 + 1 % N2) to a concentration of 2 mM. Furthermore, the cells were suspended in the culture medium prepared by adding the growth factors bFGF, FGF3, FGF10, and FGF19 such that the concentration of each growth factor was 25 ng/mL. This cell suspension was seeded in wells coated with poly-L-ornithine/fibronectin such that the cell concentration was approximately 1/3 of the cell concentration before the cell detaching treatment per well, and adhesion culture was performed under low oxygen conditions (4% of O₂, 5% of CO₂).

### (Day 10)

The culture medium was exchanged with a culture medium prepared by adding L-glutamine to a serum-free culture medium (DMEM/F12 + 2% B27 + 1% N2) such that the concentration thereof was 2 mM, and further adding growth factors bFGF, EGF, and IGF-1 thereto such that the concentrations of the growth factors were each 20 ng/mL, 20 ng/mL, and 50 ng/mL.

### [Differentiation induction method of inner ear stria vascularis marginal cells]

### (Day 11)

The differentiation into inner ear stria vascularis marginal cells was induced by the same method as after Day 12 in Test Example 2.

The expression state of various marker proteins in the cells after the differentiation induction by the above-described method was examined by immunostaining.

### [3-1] NKCC1 (Day 60)

The cells on the 49th day (Day 60) after the start of the suspension culture were treated with 4% paraformaldehyde at room temperature for 15 minutes to be fixed, and a 7 µm frozen section was prepared. The frozen section was treated with 0.3% PBST for 10 minutes and then subjected to blocking with 10% normal goat serum/0.1% PBST at room temperature for 1 hour. As the primary antibody, a rabbit anti-NKCC1 antibody was used at a concentration of 1:100 and reacted at 4°C overnight. The primary antibody was reacted with a fluorescently labeled secondary antibody specific to the IgG animal species at room temperature for 1 hour. In addition, the nucleus was stained with Hoechst 33258. FIG. 11 shows a microscopic observation image obtained with a confocal microscope (scale bar: 10 µm).

As a result, as shown in FIG. 11, the expression of NKCC1, a known functional protein in the inner ear stria vascularis marginal cells, was confirmed. Therefore, by the above method, it was confirmed that the differentiation from the inner ear progenitor cells differentiation-induced from the human iPS cells by a method different from that in Test Example 1 into inner ear stria vascularis marginal cells was induced.

### [3-2] ESRRB, KCNQ1 (Day 60)

The cells on the 49th day (Day 60) after the start of the suspension culture were treated with 4% paraformaldehyde at room temperature for 15 minutes to be fixed, and a 7 µm frozen section was prepared. The frozen section was treated with 0.3% PBST for 10 minutes and then subjected to blocking with 10% normal donkey serum/0.1% PBST at room temperature for 1 hour. As the primary antibodies, a mouse anti-ESRRB antibody and a rabbit anti-KCNQ1 antibody were used at a concentration of 1:100 each and reacted at 4°C overnight. The primary antibody was reacted with a fluorescently labeled secondary antibody specific to the IgG animal species at room temperature for 1 hour. In addition, the nucleus was stained with Hoechst 33258. FIG. 12 shows a microscopic observation image obtained with a confocal microscope (scale bar: 10 µm).

As a result, as shown in FIG. 12, the expression of ESRRB and KCNQ1, which are known functional proteins in the inner ear stria vascularis marginal cells, was detected. In addition, ESRRB was considered to be localized on the nucleus and can reproduce the expression pattern in the endogenous marginal cells.

### [3-3] ZO-1, Claudin-1, Occludin (Day 60)

The cells on the 49th day (Day 60) after the start of the suspension culture were treated with 10% TCA at 4°C for 15 minutes to be fixed, and a 7 µm frozen section was prepared. The frozen section was treated with 0.3% PBST for 10 minutes and then subjected to blocking with 10% normal goat serum/0.1% PBST at room temperature for 1 hour (ZO-1 antibody was not blocked). As the primary antibodies, a goat anti-ZO-1 antibody, a rabbit anti-Claudin-1 antibody, and a rabbit anti-Occludin antibody were used at a concentration of 1:100 each, and reacted at 4°C overnight. The primary antibody was reacted with a fluorescently labeled secondary antibody specific to the IgG animal species at room temperature for 1 hour. In addition, the nucleus was stained with Hoechst 33258. FIG. 13 shows a microscopic observation image obtained with a KEYENCE BZ-X810 microscope (scale bar: 10 µm).

As a result, as shown in FIG. 13, the expression of ZO-1, Claudin-1, and Occludin, which are known intercellular tight junction proteins in the inner ear stria vascularis marginal cells, was detected. In addition, a characteristic cobblestone-like signal was shown, and it was considered that the differentiation-induced cells were inner ear stria vascularis marginal cells which were epithelial cell-like cells.

### [Test Example 4]

In the present test example, in order to induce differentiation into the inner ear stria vascularis marginal cells from the inner ear progenitor cells, two-dimensional culture of the inner ear stria vascularis marginal cells was attempted.

### [Differentiation induction method (1)]

### (Day 20)

The inner ear progenitor cells were obtained from human iPS cells by the same method as in Test Example 1, and the inner ear progenitor cells were cultured until Day 20 by the same method as in Test Example 2.

The cells were collected, and treated at 37°C for 20 minutes using a trypsin-like enzyme preparation (trade name "TrypLE Select", Thermo Fisher Scientific, Inc.) to which EDTA was added to a concentration of 1 mM. Thereafter, 3 to 5 times the amount of DMEM/F12 was added, the cells were passed through a nylon mesh (pore size: 40 µm), and the number of cells was counted with a hemocytometer.

The cells after the detaching treatment were suspended in a culture medium having the same composition as the culture medium of Day 20 to a concentration of 1.2 × 10⁶ cells/10 mL and seeded on feeder cells pre-cultured separately in advance. The feeder cells were prepared by seeding melanocytes (trade name "Normal Human Epidermal Melanocyte", TAKARA BIO INC.) at 1 × 10⁵ cells/cm² per well of an 8-well chamber (trade name "Chamber Slide II", IWAKI & CO., LTD.) and culturing the melanocytes in an M2 culture medium for melanocyte proliferation (TAKARA BIO INC.) for 6 to 7 days, and then performing adhesion culture until the cells became confluent.

Thereafter, the timing of the culture medium exchange was also performed in the same manner using the culture medium having the same composition as that used in a case where the suspension culture was performed, and the co-culture with the feeder cells (melanocytes) was performed until the 53rd day (Day 65) after the start of the suspension culture.

### [4-1] ZO-1, Claudin-1 (Day 65)

The cells on the 53rd day (Day 65) from the start of suspension culture were treated with 10% TCA at 4°C for 15 minutes to be fixed, treated with 0.3% PBST for 10 minutes, and then subjected to blocking with 10% normal donkey serum/0.1% PBST at room temperature for 1 hour. As the primary antibodies, a goat anti-ZO-1 antibody and a rabbit anti-Claudin-1 antibody were used at a concentration of 1:100 each, and reacted at 4°C overnight. The primary antibody was reacted with a fluorescently labeled secondary antibody specific to the IgG animal species at room temperature for 1 hour. In addition, the nucleus was stained with Hoechst 33258. FIG. 14 shows a microscopic observation image obtained with a confocal microscope (scale bar: 20 µm).

As a result, as shown in FIG. 14, the expression of ZO-1 and Claudin-1, which are known intercellular tight junction proteins in the inner ear stria vascularis marginal cells, was detected. In addition, a cobblestone-like signal characteristic of the epithelial cell was shown, and it was considered that the differentiation into the inner ear stria vascularis marginal cells, which were epithelial cell-like cells, could be induced even in two-dimensional culture in which the cells were grown and extended in a single layer on the melanocytes by co-culturing with the melanocytes that had been subjected to adhesion culture in advance.

### [Differentiation induction method (2)]

### (Day 20)

The inner ear progenitor cells were obtained from human iPS cells by the same method as in Test Example 1, and the inner ear progenitor cells were cultured until Day 21 by the same method as in Test Example 2.

Thereafter, the co-culture with feeder cells (melanocytes) was performed in the same manner as in the "differentiation induction method (1)" until the 48th day (Day 60) after the start of suspension culture.

### [4-2] Occludin (Day 60)

The cells on the 48th day (Day 60) from the start of suspension culture were treated with 4% paraformaldehyde at room temperature for 15 minutes to be fixed, treated with 0.3% PBST for 10 minutes, and then subjected to blocking with 10% normal donkey serum/0.1% PBST at room temperature for 1 hour. As the primary antibody, a rabbit anti-Occludin antibody was used at a concentration of 1:100 and reacted at 4°C overnight. The primary antibody was reacted with a fluorescently labeled secondary antibody specific to the IgG animal species at room temperature for 1 hour. In addition, the nucleus was stained with Hoechst 33258. FIG. 15 shows a microscopic observation image obtained with a confocal microscope (scale bar: 10 µm).

As a result, as shown in FIG. 15, the expression of Occludin, a known intercellular tight junction protein in the inner ear stria vascularis marginal cells, was detected. In addition, a cobblestone-like signal characteristic of the epithelial cell was shown, and it was considered that the differentiation into the inner ear stria vascularis marginal cells, which were epithelial cell-like cells, could be induced even in two-dimensional culture in which the cells were grown and extended in a single layer on the melanocytes by co-culturing with the melanocytes that had been subjected to adhesion culture in advance.

### [Differentiation induction method (3)]

### (Day 20)

The inner ear progenitor cells were obtained from human iPS cells by the same method as in Test Example 1, and the inner ear progenitor cells were cultured until Day 20 by the same method as in Test Example 2.

Thereafter, the co-culture with feeder cells (melanocytes) was performed in the same manner as in the "differentiation induction method (1)" until the 53rd day (Day 65) after the start of suspension culture.

### [4-3] NKCC1, KCNQ1, LRP2 (Day 65)

The cells on the 48th day (Day 60) from the start of suspension culture were treated with 4% paraformaldehyde at 4°C overnight to be fixed, treated with 0.3% PBST for 10 minutes, and then subjected to blocking with 10% normal donkey serum/0.1% PBST at room temperature for 1 hour. As the primary antibodies, a goat anti-NKCC1 antibody, a rabbit anti-KCNQ1 antibody, and a mouse anti-LRP2 antibody were used at a concentration of 1:100 each, and reacted at 4°C overnight. The primary antibody was reacted with a fluorescently labeled secondary antibody specific to the IgG animal species at room temperature for 1 hour. In addition, the nucleus was stained with Hoechst 33258. FIG. 16 shows microscopic observation images obtained with a confocal microscope (scale bar: upper row 10 µm, lower row 5 µm).

As a result, as shown in FIG. 16, the expression of NKCC1, KCNQ1, and LRP2, which are known functional proteins in the inner ear stria vascularis marginal cells, was detected. In addition, KCNQ1 and LRP2 were co-localized on the cell membrane, while NKCC1 and KCNQ1 were not co-localized on the cell membrane, and it was considered that also in two-dimensional culture, the differentiation into cells that express the marginal cell marker in the same polarity as the endogenous marginal cells could be induced.

### [Test Example 5]

In the present test example, the effect of using an insulin-free culture medium as a culture medium in the process of differentiation induction from the inner ear progenitor cells into the inner ear stria vascularis marginal cells was examined.

### [Differentiation induction method]

### (Day 12)

The differentiation from human iPS cells into inner ear progenitor cells was induced by the same method as in Test Example 1, the cells on the 12th day (Day 12) from the start of the differentiation induction were detached off with Accutase, an equal amount of PBS was added thereto, the cells were passed through a nylon mesh (pore size: 40 µm), and the number of cells was counted with a hemocytometer.

The cells were suspended to a concentration of 5 × 10⁵ cells/10 mL and seeded in a low adhesion 6-well plate (trade name "Corning Ultra-Low Attachment Plate", Corning Inc.), and suspension culture was started under low oxygen conditions (4% of O₂, 5% of CO₂). At this time, the cells were suspended and suspension culture was started using a culture medium prepared by adding growth factors bFGF, EGF, IGF-1, FGF3, and FGF10 to a serum-free culture medium (DMEM/F12 + 2% B27 + 1% N2) such that the concentrations of the growth factors were each 10 ng/mL, 10 ng/mL, 25 ng/mL, 50 ng/mL, and 50 ng/mL, further adding L-glutamine such that the concentration was 2 mM, adding Y-27632 such that the concentration was 10 µM, adding Matrigel such that the concentration was 1%, adding SB431542 such that the concentration was 1 µM, and adding heparin such that the concentration was 50 ng/mL.

From the 8th day (Day 20) after the start of the suspension culture, the culture was performed under the following three different culture conditions.
· Condition 1) an insulin-containing serum substitute-used culture medium:
   The suspension culture was continued until Day 59 by the same method as after Day 20 of Test Example 2, except that a serum-free culture medium (DMEM/F12 + 2% B27 + 1 % N2) was used as the culture medium.
· Condition 2) an insulin-free culture medium:
   The suspension culture was continued until Day 59 by the same method as after Day 20 of Test Example 2, except that a serum-free culture medium (DMEM/F12 + 2% N21-Ins) was used as the culture medium.
· Condition 3) a culture medium obtained by adding insulin to an insulin-free culture medium:
   The suspension culture was continued until Day 59 by the same method as after Day 20 of Test Example 2, except that a culture medium in which insulin was added to a serum-free culture medium (DMEM/F12 + 2% N21-Ins) in a concentration of 1.5 µM was used as the culture medium.

### (Day 60)

In any of the above Conditions 1) to 3), the cells on the 48th day (Day 60) after the start of the suspension culture were collected, and the expression level of the OTX2 gene, which is known to be expressed on the Reissner's membrane, which is an inner ear organ, and known to be poorly expressed in the inner ear stria vascularis marginal cells, was quantified by qPCR in the same manner as in Test Example 1.

As a result, as shown in FIG. 17, it was found that the expression level of OTX2 was low in a case where the culture medium did not contain insulin as compared with a case where insulin was contained in the culture medium (an insulin-containing serum substitute-used culture medium and a culture medium obtained by adding insulin to the insulin-free culture medium). That is, the expression level of OTX2 could be reduced by not adding insulin to the culture medium. As a result, it was considered that it is necessary to use an insulin-free culture medium in order to obtain cells that exhibit the same inherent physiological functionality as the endogenous marginal cells.

### [Test Example 6]

The inner ear progenitor cells were obtained from human iPS cells by the same method as in Test Example 1, and suspension culture was continued until the 49th day (Day 61) after the start of the suspension culture by the same method as in Test Example 2 using the inner ear progenitor cells.

For the obtained cells, a barrier function assay was performed. Specifically, the culture after the suspension culture was put into a microtube having a volume of 1.5 mL for each cell, and centrifuged, the culture medium was discarded, and 0.5 mL of HBSS containing 2 mM EDTA was added. In addition, as a control, a sample to which HBBS containing no EDTA was added was also prepared under the same conditions. Each of the samples was placed on ice for 15 minutes, and then 4 kDa FITC-Dextran was added to each of the samples to a concentration of 2 mg/mL, and the samples were suspended and immediately observed with a confocal microscope.

As a result, as shown in the upper row of FIG. 18, a fluorescence image showing that FITC-Dextran (fluorescence) was transferred to the inside of the cell mass was not observed in the case of the EDTA non-treatment, whereas as shown in the lower row of FIG. 18, a fluorescence image showing that FITC-Dextran (fluorescence) was transferred to the inside of the cell mass was observed in the case of the EDTA treatment. This is considered to be because, in the case of the EDTA non-treatment, the barrier function between cells was exhibited and the cells were not transferred into the inside of the cell mass, whereas in the case of the EDTA treatment, the calcium-dependent barrier function was impaired and the cells were transferred into the inside of the cell mass.

From the above, it was clarified that, according to the method of the present invention, cells that exhibit the same inherent physiological functionality as the stria vascularis marginal cells that are endogenous to the inner ear organ could be obtained.

### [Test Example 7]

In the present test example, the agent assessment was performed using the inner ear stria vascularis marginal cells differentiation-induced by the same method as in Test Example 3.

Specifically, on the 47th day (Day 58) after the start of the suspension culture in Test Example 3, 10 µM of cisplatin (anticancer agent) or 100 µM of neomycin (aminoglycoside-based antibiotic) was added to the culture medium, and the suspension culture was continued for 48 hours until Day 60 under normal oxygen conditions (20% of O₂, 5% of CO₂).

The expression state of various marker proteins in the cells after the agent treatment by the above-described method was examined by immunostaining.

### [7-1] Cleaved caspase-3, ZO-1 (Day 60)

The cells on the 49th day (Day 60) after the start of the suspension culture and on the 2nd day after the start of the agent treatment were treated with 4% paraformaldehyde at room temperature for 30 minutes to be fixed, and a 7 µm frozen section was prepared. In addition, as a control, a frozen section was similarly prepared using the cells on the 49th day (Day 60) after the start of the suspension culture without agent treatment. Each of the frozen sections was treated with 0.3% PBST for 10 minutes and then subjected to blocking with 10% normal donkey serum/0.1 % PBST at room temperature for 1 hour. As the primary antibody, a rabbit anti-cleaved caspase-3 antibody and a goat anti-ZO-1 antibody were used at a concentration of 1:200 each, and reacted at 4°C overnight. The primary antibody was reacted with a fluorescently labeled secondary antibody specific to the IgG animal species at room temperature for 1 hour. In addition, the nucleus was stained with Hoechst 33258. FIG. 19 shows a microscopic observation image obtained with a confocal microscope (scale bar: 20 µm).

Furthermore, in each of the microscopic observation images, the total number of cells was counted with Hoechst 33258, the number of cells expressing cleaved caspase-3 was counted, and the proportion of the cells expressing cleaved caspase-3 to the total number of cells was calculated. FIG. 20 shows the results.

As a result, as shown in FIGS. 19 and 20, it was confirmed that in a case where the treatment was performed with each agent, the number of cells expressing cleaved caspase-3, a known marker of apoptosis, was increased as compared with the control. From the above, it was clarified that ototoxicity of various agents could be assessed by using the inner ear stria vascularis marginal cells obtained by inducing differentiation from human iPS cells.

## Claims

1. A method for producing inner ear stria vascularis marginal cells, the method comprising:
a step of culturing a cell population including inner ear progenitor cells differentiation-induced from pluripotent stem cells in an insulin-free culture medium that does not contain insulin or contains insulin only in a trace amount.

2. The method for producing inner ear stria vascularis marginal cells according to Claim 1,
wherein the insulin-free culture medium has an insulin concentration of 0 nM or more and 100 nM or less.

3. The method for producing inner ear stria vascularis marginal cells according to Claim 1 or 2,
wherein the insulin-free culture medium contains EGF and further contains at least one or more selected from the group consisting of bFGF, FGF3, and BMP4.

4. The method for producing stria vascularis marginal cells according to Claim 3,
wherein the insulin-free culture medium is a culture medium in which FGF3 and BMP4 are selected.

5. The method for producing inner ear stria vascularis marginal cells according to Claim 1,
wherein the method for producing inner ear stria vascularis marginal cells includes the following steps (1) and (2),
(1) a step of subjecting the cell population including the inner ear progenitor cells differentiation-induced from the pluripotent stem cells to cell detaching and dispersing treatment,
(2) a step of subjecting the cells or the cell population obtained in the step (1) to suspension culture in the insulin-free culture medium in a presence of an extracellular matrix material.

6. The method for producing inner ear stria vascularis marginal cells according to Claim 5,
wherein the extracellular matrix material includes at least one or more selected from the group consisting of Matrigel, Pronectin, collagen, laminin, and fibronectin.

7. The method for producing inner ear stria vascularis marginal cells according to Claim 1,
wherein the method for producing stria vascularis marginal cells includes the following steps (1) to (3),
(1) a step of subjecting the cell population including the inner ear progenitor cells differentiation-induced from the pluripotent stem cells to cell detaching and dispersing treatment,
(2) a step of subjecting the cells or the cell population obtained in the step (1) to suspension culture in the insulin-free culture medium in a presence of an extracellular matrix material,
(3) a step of seeding the cells or the cell population after the suspension culture in the step (2) on feeder cells that have been separately subjected to adhesion culture in advance, and culturing the cells or the cell population in the insulin-free culture medium.

8. The method for producing inner ear stria vascularis marginal cells according to Claim 7,
wherein the feeder cells are melanocytes or melanocyte-like cells.

9. The method for producing inner ear stria vascularis marginal cells according to Claim 1,
wherein the cell population including the inner ear progenitor cells differentiation-induced from the pluripotent stem cells is obtained by a method including the following steps (1) to (4),
(1) a step of culturing the pluripotent stem cells in an absence of a growth factor and in a presence of a ROCK inhibitor,
(2) a step of culturing the cell population obtained in the step (1) in the absence of a growth factor and a ROCK inhibitor,
(3) a step of culturing the cell population obtained in the step (2) in the presence of at least one growth factor selected from the group consisting of bFGF, FGF3, FGF10, and FGF19, and in the presence of BMP4,
(4) a step of culturing the cell population obtained in the step (3) in the presence of at least one growth factor selected from the group consisting of bFGF, FGF3, FGF10, and FGF19, and in the absence of BMP4.

10. The method for producing inner ear stria vascularis marginal cells according to Claim 1,
wherein the inner ear stria vascularis marginal cells are obtained under a serum-free condition.

11. The method for producing inner ear stria vascularis marginal cells according to Claim 1,
wherein the inner ear stria vascularis marginal cells express a potassium channel protein and a tight junction protein.

12. An agent assessment method, comprising:
a step of treating inner ear stria vascularis marginal cells that are obtained by inducing differentiation from inner ear progenitor cells with a test agent; and
a step of assessing a state of the inner ear stria vascularis marginal cells treated with the test agent.

13. A cell culture for assessing an agent, comprising:
inner ear stria vascularis marginal cells that are obtained by inducing differentiation from inner ear progenitor cells.

14. A method for producing inner ear stria vascularis marginal cells, the method comprising:
a step of culturing a cell population including inner ear progenitor cells in an insulin-free culture medium that does not contain insulin or contains insulin only in a trace amount.
